# FASCICULE DE BREVET EUROPEEN

(11) **EP 4 433 101 B1**
(45) Date de publication et mention de la délivrance du brevet: **22.10.2025**
(21) Numéro de dépôt: 22818661.5
(22) Date de dépôt: 17.11.2022
(51) Int. Cl.: A61L 9/02, A61L 9/03, A61L 9/12, A01M 1/20, A01M 1/02

(54) **APPAREIL DIFFUSEUR DESTINÉ À DISPERSER DANS L'AIR, À L'ÉTAT DE VAPEUR, UNE SUBSTANCE À L'ÉTAT LIQUIDE OU SOLIDE À TEMPÉRATURE AMBIANTE**
DIFFUSORVORRICHTUNG ZUM DISPERGIEREN EINER BEI RAUMTEMPERATUR FLÜSSIGEN ODER FESTEN SUBSTANZ IN DIE LUFT ALS DAMPF
DIFFUSER DEVICE FOR DISPERSING A SUBSTANCE THAT IS IN THE LIQUID OR SOLID STATE AT ROOM TEMPERATURE INTO THE AIR AS VAPOR

(30) Priorité: 17.11.2021 FR 2112164
(43) Date de publication de la demande: 25.09.2024
(73) Titulaire: CAELIMP, 31670 Labege (FR)
(72) Inventeur: DUFOUR, Samuel, 31400 TOULOUSE (FR); JOURDAN, Arnaud, 31100 TOULOUSE (FR); RIVIERE, Philippe, 31500 TOULOUSE (FR)
(74) Mandataire: Loyer & Abello
(86) Numéro de dépôt international: PCT/EP2022/082258
(87) Numéro de publication internationale: WO 2023/089019

(56) Documents cités:
- US-A1- 2008 290 186
- US-A1- 2017 072 085
- US-A1- 2021 276 028

## Description

### Domaine technique

L'invention se rapporte au domaine des appareils diffuseurs destinés à disperser dans l'air, à l'état de vapeur, une substance à l'état liquide ou solide à température ambiante.

### Technique antérieure

Un appareil diffuseur du type précité est connu par exemple par le document WO 2019/243734 A1. Dans cet appareil, un récipient de stockage contenant la substance est relié à un organe distributeur. L'organe distributeur comporte des micro-canalisations formant une sortie agencée dans la canalisation afin d'y constituer une zone d'évaporation de la substance. Un organe de chauffage est agencé sur ou dans l'organe distributeur de manière à contrôler un écoulement de la substance à travers l'organe distributeur. Un ventilateur génère un débit d'air balayant la surface d'évaporation. Dans des modes de réalisation, l'organe distributeur est en dessous du récipient de stockage suivant la direction de l'accélération de la pesanteur, et le débit d'air généré par le ventilateur balaye la surface d'évaporation dans la direction de l'accélération de la pesanteur. D'autres appareils diffuseurs de ce type sont connu de US 2021/276028 A1 et US 2008/290186 A1.

### Résumé de l'invention

L'invention propose un appareil diffuseur pour disperser dans l'air, à l'état de vapeur, une substance à l'état liquide ou solide à température ambiante, l'appareil diffuseur comportant :
- un réceptacle ;
- un récipient de stockage contenant la substance et présentant un orifice de vidange, le récipient de stockage étant reçu dans le réceptacle de sorte que l'orifice de vidange est orienté dans une direction descendante de l'appareil diffuseur ;
- un organe distributeur positionné en sortie de l'orifice de vidange et relié à l'orifice de vidange, l'organe distributeur comprenant un corps poreux présentant une surface d'évaporation et étant situé au-delà de l'orifice de vidange suivant la direction descendante de l'appareil diffuseur ; le récipient de stockage étant reçu dans le réceptacle de sorte que l'orifice de vidange est orienté vers le corps poreux suivant une direction descendante de l'appareil diffuseur;
- un organe de chauffage agencé sur ou dans le corps poreux de manière à contrôler un écoulement de la substance à travers le corps poreux ; et
- un système d'aération comprenant une entrée d'air, au moins un ventilateur et au moins une sortie d'air, le système d'aération étant configuré pour créer un débit d'air allant depuis l'entrée d'air vers ladite au moins une sortie d'air en balayant la surface d'évaporation dans une direction montante de l'appareil diffuseur opposée à la direction descendante. La substance présente une viscosité variable en fonction de la température, ladite viscosité étant telle que, dans la position d'utilisation, la substance ne peut pas s'écouler à travers le corps poreux à toute température ambiante inférieure à une première température, la première température étant supérieure à 0°C, et que la substance s'écoule à travers le corps poreux à une deuxième température supérieure à la première température.

L'appareil diffuseur est destiné à être utilisé dans une position d'utilisation dans laquelle la direction descendante est vers le bas et la direction montante est vers le haut par rapport à l'accélération de la pesanteur. Dans la position d'utilisation, l'accélération de pesanteur constitue une force motrice susceptible de contribuer à générer un écoulement de la substance depuis l'orifice de vidange vers la surface d'évaporation, donc dans la direction descendante.

L'organe de chauffage permet de contrôler cet écoulement de la substance à travers le corps poreux, simplement en chauffant ou non le corps poreux. Les principes physiques à la base de ce contrôle de l'écoulement de la substance à travers le corps poreux sont décrits dans le document WO 2019/243734 A1 déjà cité précédemment ou encore dans le document WO 2020/254733 A1.

Comme décrit dans ces documents, la chaleur fournie par l'organe de chauffage abaisse la viscosité dynamique de la substance, ce qui permet au fluide de circuler au sein de l'organe distributeur selon la loi de Darcy puis de s'étaler en surface dudit organe distributeur. Sans apport de chaleur, la circulation est figée car la somme des adhérences au sein de l'organe distributeur suit la loi de Jurin. En d'autres termes, l'écoulement est permis à travers l'organe distributeur à chaud mais stoppé à température ambiante par la force d'adhérence entre le fluide et la surface de l'organe distributeur.

Pour permettre l'écoulement à travers l'organe distributeur de cette façon, il faut chauffer l'organe distributeur jusqu'à atteindre une température de consigne. La température de consigne peut être choisie de manière que l'écoulement de la substance s'effectue à un débit suffisamment faible pour éviter la formation de gouttes se détachant de l'organe distributeur (phénomène désigné ci-après par le terme « gouttage ») lorsque l'organe distributeur est à la température de consigne.

Mais avant que l'organe distributeur n'atteigne l'équilibre thermique à la température de consigne, l'organe distributeur est dans un régime transitoire, qui peut durer de l'ordre de 5 à 20 minutes environ. La demanderesse a constaté que même si la température de consigne est choisie pour éviter le gouttage à l'équilibre thermique, le gouttage peut néanmoins se produire pendant le régime transitoire. Or le gouttage est indésirable, car la substance qui a ainsi goutté n'est pas évaporée comme souhaité au niveau de l'organe distributeur, et peut même être gaspillée si elle s'échappe de l'appareil diffuseur. Ceci est particulièrement indésirable si l'appareil diffuseur est utilisé pendant des durées courtes, par exemple de l'ordre d'une à quelques heures consécutives, car alors la durée du régime transitoire n'est pas négligeable par rapport à la durée d'utilisation de l'appareil diffuseur.

La demanderesse a constaté que lorsque le système d'aération est configuré pour que le débit d'air balaye la surface d'évaporation dans la direction montante, donc dans la direction opposée à la direction descendante, le débit d'air tend à empêcher l'accumulation de substance liquide au niveau d'une partie inférieure du corps poreux suivant la direction descendante. Ce choix de direction du débit d'air permet ainsi de supprimer ou à tout le moins limiter le gouttage, même dans le régime transitoire.

Une idée à la base de certains modes de réalisation de l'invention consiste à proposer un agencement de l'organe distributeur et du récipient de stockage qui facilite la mise en place du récipient de stockage dans l'appareil diffuseur.

L'invention propose ainsi aussi un appareil diffuseur pour disperser dans l'air, à l'état de vapeur, une substance à l'état liquide ou solide à température ambiante, l'appareil diffuseur comportant :
- un réceptacle débouchant à l'air libre par une ouverture orientée dans une direction montante de l'appareil diffuseur, c'est-à-dire par exemple orientée vers le haut lorsque l'appareil diffuseur est dans une position d'utilisation ;
- un récipient de stockage contenant la substance et présentant un orifice de vidange, le récipient de stockage étant reçu dans le réceptacle de sorte que l'orifice de vidange est orienté dans une direction descendante de l'appareil diffuseur, c'est-à-dire par exemple vers le bas dans la position d'utilisation ;
- un organe distributeur positionné en sortie de l'orifice de vidange et relié à l'orifice de vidange, l'organe distributeur comprenant un corps poreux présentant une surface d'évaporation située dans le réceptacle, notamment dans la position d'utilisation, et étant situé au-delà de l'orifice de vidange suivant la direction descendante de l'appareil diffuseur, c'est-à-dire par exemple en dessous de l'orifice de vidange dans la position d'utilisation ;
- un organe de chauffage agencé sur ou dans le corps poreux de manière à contrôler un écoulement de la substance à travers le corps poreux ; et
- un système d'aération comprenant une entrée d'air et au moins un ventilateur, le système d'évaporation étant configuré pour créer un débit d'air allant depuis l'entrée d'air vers l'ouverture en balayant la surface d'évaporation.

Dans cet appareil diffuseur, l'ouverture par laquelle le réceptacle débouche à l'air libre forme la sortie d'air du système d'aération.

Dans cet appareil diffuseur également, l'organe de chauffage permet de contrôler l'écoulement de la substance à travers le corps poreux, simplement en chauffant ou non le corps poreux, comme déjà mentionné ci-dessus.

Grâce au fait que le réceptacle débouche à l'air libre par une ouverture orientée dans une direction montante de l'appareil diffuseur, c'est-à-dire par exemple orientée vers le haut lorsque l'appareil diffuseur est dans une position d'utilisation, il est aisé de mettre en place le récipient de stockage en place dans l'appareil diffuseur, puisqu'il suffit d'insérer le récipient de stockage dans le réceptacle par l'ouverture.

Un tel appareil diffuseur trouve une application particulière pour diffuser une substance dans un lieu clos tel qu'une serre ou un bâtiment, par exemple, ou dans un lieu à l'abri des précipitations. En effet, dans de tels lieux, il n'est pas problématique que l'ouverture soit orientée dans une direction montante de l'appareil diffuseur, c'est-à-dire par exemple orientée vers le haut lorsque l'appareil diffuseur est dans la position d'utilisation, puisque l'eau venant de précipitations ne risque pas d'entrer dans l'appareil diffuseur par cette ouverture.

D'autre part, puisque le système d'aération est configuré pour créer un débit d'air allant depuis l'entrée d'air vers l'ouverture en balayant la surface d'évaporation, l'appareil diffuseur permet d'obtenir une bonne dispersion d'air chargé en substance évaporée dans l'air ambiant.

D'autre part, dans cet appareil diffuseur également, le choix de direction du débit d'air permet de supprimer ou à tout le moins limiter le gouttage, même dans le régime transitoire.

Selon des modes de réalisation, l'un ou l'autre des appareils diffuseurs décrits ci-dessus peut présenter une ou plusieurs des caractéristiques suivantes.

Selon un mode de réalisation, le réceptacle comporte une paroi de séparation interne, la paroi de séparation interne entourant au moins partiellement et de préférence entièrement le récipient de stockage lorsque le récipient de stockage est reçu dans le réceptacle, et la paroi de séparation interne étant configurée de sorte que le débit d'air circule autour de la paroi de séparation interne.

Avec une telle paroi de séparation interne, s'il se produit une recondensation de la substance évaporée dans le réceptacle, cette recondensation tend à se produire sur la paroi de séparation interne plutôt que sur le récipient de stockage.

Selon un mode de réalisation, l'appareil diffuseur comporte un boîtier, le boîtier comportant une paroi supérieure et une paroi latérale, et l'ouverture débouche sur la paroi supérieure.

Selon un mode de réalisation, le boîtier comporte une paroi interne définissant le réceptacle et s'étendant depuis la paroi supérieure.

Selon un mode de réalisation, la paroi supérieure et la paroi latérale sont formées dans une portion supérieure du boîtier, la portion supérieure étant montée de façon coulissante de façon à pouvoir coulisser par rapport à la paroi latérale suivant la direction descendante de l'appareil diffuseur, entre :
- une position relevée dans laquelle une paroi supérieure du récipient de stockage est située au-delà de la paroi supérieure suivant la direction descendante de l'appareil diffuseur, c'est-à-dire par exemple en-dessous de la paroi supérieure, lorsque le récipient de stockage est reçu dans le réceptacle ; et
- une position abaissée dans laquelle la paroi supérieure du récipient de stockage est située au-delà de la paroi supérieure suivant la direction montante de l'appareil diffuseur, c'est-à-dire par exemple au-dessus de la paroi supérieure du récipient de stockage, lorsque le récipient de stockage est reçu dans le réceptacle.

Selon un mode de réalisation, le corps poreux présente une porosité dans une partie intérieure du corps poreux plus faible qu'une porosité dans une partie extérieure du corps poreux entourant la partie intérieure.

Selon un mode de réalisation, le corps poreux comporte une mèche en bois, en textile, en céramique, en polymère, ou en un matériau métallique poreux obtenu par frittage d'une poudre métallique ou d'une poudre d'alliage métallique.

Selon un mode de réalisation, l'organe distributeur est solidaire du récipient de stockage.

Ainsi, le récipient de stockage et l'organe distributeur peuvent être fournis sous la forme d'un ensemble amovible à insérer d'un seul tenant dans le réceptacle. Ceci rend encore plus aisée l'utilisation de l'appareil diffuseur, puisqu'il suffit d'insérer cet ensemble amovible dans le réceptacle par l'ouverture. Toutefois, d'autres agencements sont possibles. Ainsi, dans un mode de réalisation, l'organe distributeur ou à tout le moins le corps poreux est à demeure dans l'appareil diffuseur. Dans un autre mode de réalisation, l'organe distributeur peut être démonté du récipient de stockage.

Selon un mode de réalisation, l'orifice de vidange est obturé par un opercule, et l'appareil diffuseur comprend en outre un dispositif de perforation disposé entre le corps poreux et l'opercule, le dispositif de perforation comprenant une pluralité de dents et/ou d'aiguilles configurées pour perforer l'opercule lorsque le corps poreux est déplacé en direction de l'opercule.

Selon un mode de réalisation, le réceptacle et le corps poreux sont conformés de telle sorte que ledit déplacement du corps poreux en direction de l'opercule se produit lors de l'installation de l'ensemble amovible dans le réceptacle.

Selon un mode de réalisation, l'orifice de vidange est disposé à une première extrémité du récipient de stockage, et le récipient de stockage présente, à une deuxième extrémité opposée à la première extrémité, une portion divergente facilitant la préhension du récipient de stockage et disposée au-delà de l'ouverture suivant la direction montante de l'appareil diffuseur, c'est-à-dire par exemple au-dessus de l'ouverture de façon à dévier le flux d'air sortant de l'ouverture vers une périphérie de l'appareil diffuseur.

Ceci peut tendre à davantage disperser l'air chargé en substance évaporée dans l'air ambiant.

Selon un mode de réalisation, la portion divergente affleure la paroi supérieure du boîtier ou est au-delà de la paroi supérieure suivant la direction descendante de l'appareil diffuseur, c'est-à-dire par exemple en-dessous de la paroi supérieure du boîtier, et la portion divergente est conformée de façon à dévier le flux d'air sortant de l'ouverture vers une périphérie de l'appareil diffuseur.

Selon un mode de réalisation, le boîtier comporte une extrémité inférieure opposée à la paroi supérieure du boîtier, et cette extrémité inférieure présente une surface plane. Ainsi, l'appareil diffuseur peut être posé sur une surface plane et horizontale, par exemple sur la surface d'une table ou plus généralement d'un meuble.

Divers positionnements sont possibles pour l'entrée d'air. Selon un mode de réalisation, l'entrée d'air est sur la paroi latérale du boîtier. Selon un mode de réalisation, l'entrée d'air est au-delà du corps poreux suivant la direction descendante de l'appareil diffuseur, c'est-à-dire par exemple en dessous du corps poreux dans la position d'utilisation. En particulier, le boîtier comporte une extrémité inférieure opposée à la paroi supérieure du boîtier et au-delà du corps poreux suivant la direction descendante de l'appareil diffuseur, c'est-à-dire par exemple en dessous du corps poreux dans la position d'utilisation, et l'entrée d'air est située à cette extrémité inférieure.

Selon un mode de réalisation, le ventilateur est en aval de l'entrée d'air et en amont du corps poreux suivant un sens de circulation du débit d'air. Ainsi, la substance évaporée au niveau de la surface d'évaporation ne traverse pas le ventilateur.

Divers positionnements sont possibles pour le ventilateur. Selon un mode de réalisation, le ventilateur est disposé au-delà du corps poreux suivant la direction descendante de l'appareil diffuseur, c'est-à-dire par exemple en dessous du corps poreux dans la position d'utilisation. Selon un mode de réalisation, le ventilateur est disposé entre la paroi latérale du boîtier et la paroi interne du boîtier.

Selon un mode de réalisation, le ventilateur est un ventilateur axial ou un ventilateur centrifuge.

Selon un mode de réalisation, le système d'aération comprend en outre un organe de guidage d'air, l'organe de guidage d'air comportant au moins un trou traversant en regard de la surface d'évaporation.

Selon un mode de réalisation, l'organe de guidage d'air est disposé sous le corps poreux dans la position d'utilisation.

Selon un mode de réalisation, l'organe de guidage d'air comporte une pluralité de trous traversants en regard de la surface d'évaporation.

Selon un mode de réalisation, les trous traversants présentent une section circulaire ou ovoïde avec un diamètre intérieur compris entre 0,1 mm et 4 mm.

Selon un mode de réalisation, l'organe de chauffage comprend une résistance électrique placée directement sur une surface extérieure du corps poreux ou reçue en partie dans un évidement, de préférence aveugle, que présente le corps poreux.

Selon un mode de réalisation, l'organe de chauffage s'étend à travers l'organe de guidage d'air.

Selon un mode de réalisation, l'organe de chauffage et/ou l'organe de guidage d'air comporte une dépression située au-delà d'une surface inférieure du corps poreux suivant la direction descendante de l'appareil diffuseur, c'est-à-dire par exemple en dessous de la surface inférieure du corps poreux dans la position d'utilisation, la dépression présentant une concavité tournée vers le corps poreux, par exemple tournée vers la surface inférieure du corps poreux.

Avec une telle dépression, s'il se produit un gouttage de la substance à l'état liquide, la substance ayant ainsi goutté s'accumule dans la dépression.

Selon un mode de réalisation, l'appareil diffuseur comprend en outre une carte électronique, la résistance électrique étant alimentée électriquement par la carte électronique.

Selon un mode de réalisation, l'appareil diffuseur comprend en outre un dispositif de commande configuré pour commander l'organe de chauffage en fonction d'une température de consigne dans le corps poreux.

Selon un mode de réalisation, le dispositif de commande est agencé sur la carte électronique.

Selon un mode de réalisation, le dispositif de commande est en outre configuré pour commander une vitesse de fonctionnement du ventilateur.

Selon un mode de réalisation, ladite substance comporte au moins un composé sélectionné parmi les molécules sémiochimiques, les phéromones, les allomones, les kairomones, les synomones d'origine naturelle ou synthétique.

Selon un mode de réalisation, la substance est une solution renfermant au moins une phéromone sexuelle ou non, une allomone, une synomone ou une kairomone destinée à provoquer une réponse positive ou négative relativement à l'espèce visée, dont le résultat comportemental peut être une confusion sexuelle, une confusion d'autre nature, une attraction sexuelle, une attraction d'autre nature, une répulsion de toute nature, chez les arthropodes, dont les arachnides, ou dont les hexapodes, parmi lesquels notamment les insectes, dont les insectes nuisibles.

Selon un mode de réalisation, la substance est une solution renfermant au moins une phéromone ou une phéromone sexuelle, une allomone, une synomone ou une kairomone destinée à provoquer une réponse positive ou négative relativement à l'espèce visée, dont le résultat comportemental peut être notamment un apaisement, une relaxation, une euphorisation ou une intimidation chez les classes mammalia et aves.

Selon un mode de réalisation, la substance comprend un solvant choisi parmi le myristate d'isopropyle, le dipropylène glycol, Éther de dipropylène glycol monomé-thylique, et un hydrocarbure isoparaffinique, par exemple une isoparaffine L ou P ou N ou V.

Selon un mode de réalisation, la substance comporte au moins un composé pris dans le groupe formé par les agents odorifères utilisables pour l'homme ou l'animal, les substances sémiochimiques, les agents cosmétiques, les huiles essentielles, les parfums, les agents désinfectants, les agents neutralisants d'odeur et les agents phytosanitaires et agricoles. Selon un mode de réalisation, la substance est une solution comportant au moins un composé pris dans ce groupe.

Selon un mode de réalisation, la substance comporte au moins un composé pris dans le groupe formé par les agents odorifères utilisables pour l'homme ou l'animal, les substances sémiochimiques, les agents cosmétiques, les huiles essentielles, les parfums, les agents désinfectants et les agents phytosanitaires et agricoles. Selon un mode de réalisation, la substance est une solution comportant au moins un composé pris dans ce groupe.

Selon un mode de réalisation, la substance comporte au moins un composé pris dans le groupe formé par les agents odorifères utilisables pour l'homme, les agents cosmétiques, les huiles essentielles, les parfums, les agents désinfectants, les agents neutralisants d'odeur. Selon un mode de réalisation, la substance est une solution comportant au moins un composé pris dans ce groupe.

Selon un mode de réalisation, la substance comporte au moins un composé pris dans le groupe formé par les agents odorifères utilisables pour l'homme, les agents cosmétiques, les huiles essentielles, les parfums, les agents désinfectants. Selon un mode de réalisation, la substance est une solution comportant au moins un composé pris dans ce groupe.

Selon un mode de réalisation, les agents odorifères utilisables pour l'animal sont choisis parmi les acides gras ou la forme estérifiée desdits acides gras telle que l'oléate de méthyle, le palmitate de méthyle, l'azélate de diméthyle, et le pimélate de diméthyle.

Selon un mode de réalisation, la substance présente à l'état liquide une viscosité supérieure à 1 cPa.s à 25°C, par exemple supérieure à 8 cPa.s à 25°C, et inférieure à 1 cPa.s à 60°C.

Selon un mode de réalisation, la substance présente à la pression atmosphérique une température d'ébullition comprise entre 30°C et 400°C.

Selon un mode de réalisation, la substance est à l'état liquide à température ambiante, et ledit récipient de stockage contient en outre un organe de rétention alvéolaire interne imprégné de ladite substance à l'état liquide.

Selon un mode de réalisation l'organe de rétention alvéolaire comprend un matériau choisi parmi un feutre, par exemple feutre en laine, et une mousse de mélamine.

Selon un mode de réalisation, une pluralité d'organes de rétention alvéolaires sont en contact et disposés dans le récipient de stockage.

Selon un mode de réalisation, le récipient de stockage ne présente pas d'autre ouverture que l'orifice de vidange, et le récipient de stockage contient, outre la substance, une phase gazeuse occupant au moins 20% du volume du récipient de stockage.

La première température peut être fixée dans différentes plages. Si l'appareil diffuseur est destiné à être utilisé en plein air, la première température sera notamment choisie en fonction des données climatiques locales. Selon des modes de réalisation, la première température est par exemple comprise entre 1°C et 50°C, ou entre 5°C et 40°C ou entre 10°C et 35°C, ou encore entre 15°C et 25°C.

La substance présente une viscosité variable en fonction de la température, ladite viscosité étant telle que, dans la position d'utilisation, la substance ne peut pas s'écouler à travers le corps poreux à toute température ambiante inférieure à une première température, la première température étant supérieure à 0°C, et que la substance s'écoule à travers le corps poreux à une deuxième température supérieure à la première température.

Selon un mode de réalisation, la substance est à l'état liquide à température ambiante. Par exemple la substance peut présenter à la pression atmosphérique une température de fusion comprise entre -70°C et 0°C.

Selon un mode de réalisation, la substance est à l'état solide à température ambiante. Par exemple la substance peut présenter à la pression atmosphérique une température de fusion supérieure à 30°C, par exemple comprise entre 30°C et 40°C.

Selon un mode de réalisation, l'appareil diffuseur comporte plusieurs récipients de stockage contenant des substances différentes, chacun des récipients de stockage présentant un orifice de vidange et étant reçu dans le réceptacle de sorte que l'orifice de vidange soit orienté dans une direction descendante de l'appareil diffuseur, c'est-à-dire par exemple orienté vers le bas dans la position d'utilisation. De tels récipients de stockage peuvent être réalisés de différentes manières, par exemple avec un cloisonnement intérieur d'un unique réservoir ou au moyen de plusieurs réservoirs distincts.

L'invention concerne aussi l'utilisation d'un appareil diffuseur tel que décrit précédemment pour disperser dans l'air, à l'état de vapeur, une substance à l'état liquide ou solide à température ambiante, dans laquelle l'appareil diffuseur est dans la position d'utilisation, la direction descendante de l'appareil diffuseur étant vers le bas par rapport à l'accélération de pesanteur.

Selon un mode de réalisation, l'appareil diffuseur est situé dans un lieu clos, tel qu'une serre ou un bâtiment, ou dans un lieu à l'abri des précipitations.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
[Fig.1] La [Fig.1] est une vue en perspective d'un appareil diffuseur selon une première variante de réalisation.
[Fig.2] La [Fig.2] est une autre vue en perspective de l'appareil diffuseur de la [Fig.1], où un ensemble amovible est reçu dans le réceptacle de l'appareil diffuseur.
[Fig.3A] La [Fig.3A] est une vue en perspective de l'ensemble amovible destiné à être reçu dans le réceptacle de l'appareil diffuseur.
[Fig.3B] La [Fig.3B] est une autre vue en perspective de l'ensemble amovible de la [Fig.3A].
[Fig.4A] La [Fig.4A] est une vue du récipient de stockage de l'ensemble amovible des figures 3A et 3B, en coupe plane selon IV-IV sur la [Fig.3A].
[Fig.4B] La [Fig.4B] est une vue en coupe plane analogue à la [Fig.4A], montrant le récipient de stockage et l'organe distributeur de l'ensemble amovible des figures 3A et 3B.
[Fig.4C] La [Fig.4C] est une vue agrandie, partiellement en perspective et partiellement en coupe, du détail IVC de la [Fig.4B], montrant une variante de réalisation de l'ensemble amovible.
[Fig.5A] La [Fig.5A] est une vue en coupe plane selon V-V de la [Fig.2].
[Fig.5B] La [Fig.5B] est une autre vue en coupe, en perspective, selon V-V de la [Fig.2].
[Fig.5C] La [Fig.5C] est une vue en coupe plane analogue à la [Fig.5A], montrant une façon possible d'utiliser l'appareil diffuseur.
[Fig.6] La [Fig.6] est une vue en perspective d'un appareil diffuseur selon une deuxième variante de réalisation.
[Fig.7] La [Fig.7] est une vue en coupe, en perspective, selon VII-VII de la [Fig.6].
[Fig.8] La [Fig.8] est une vue en coupe d'un appareil diffuseur selon une troisième variante de réalisation.
[Fig.9] La [Fig.9] est une vue schématique en coupe plane d'un appareil diffuseur selon une quatrième variante de réalisation.
[Fig.10] La [Fig.10] est une vue schématique en coupe plane d'un appareil diffuseur selon une cinquième variante de réalisation.
[Fig.11] La [Fig.11] est une vue schématique en coupe plane d'une variante d'exécution de l'organe de chauffage de l'appareil diffuseur.

### Description des modes de réalisation

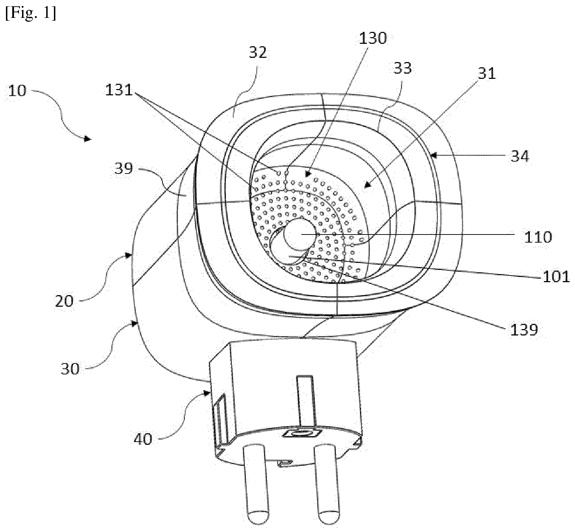

La [Fig.1] est une vue en perspective d'une première variante de réalisation d'un appareil diffuseur destiné à disperser dans l'air, à l'état de vapeur, une substance à l'état liquide à température ambiante. Cet appareil diffuseur porte la référence 10 sur les dessins ; il sera désigné ci-après « l'appareil 10 » par commodité.

L'appareil 10 comporte une partie fixe généralement désignée par 20.

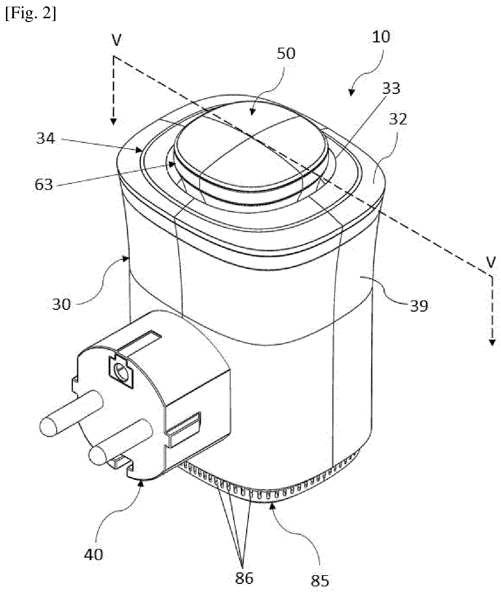

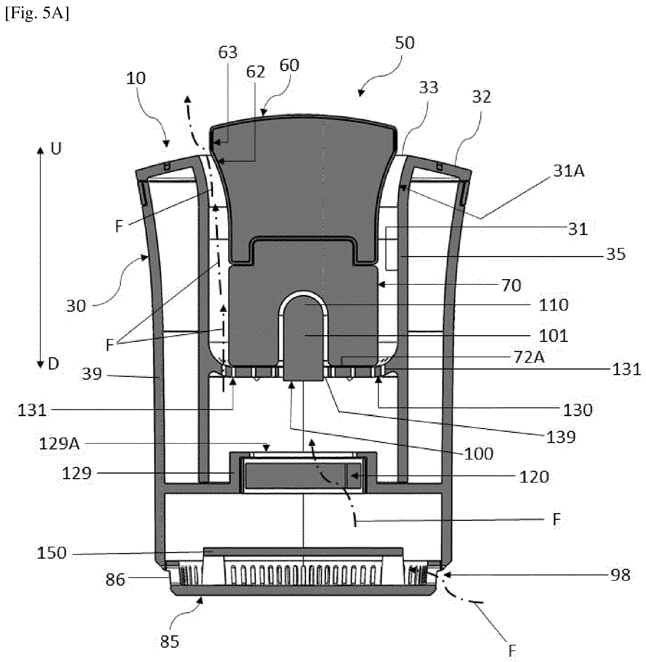

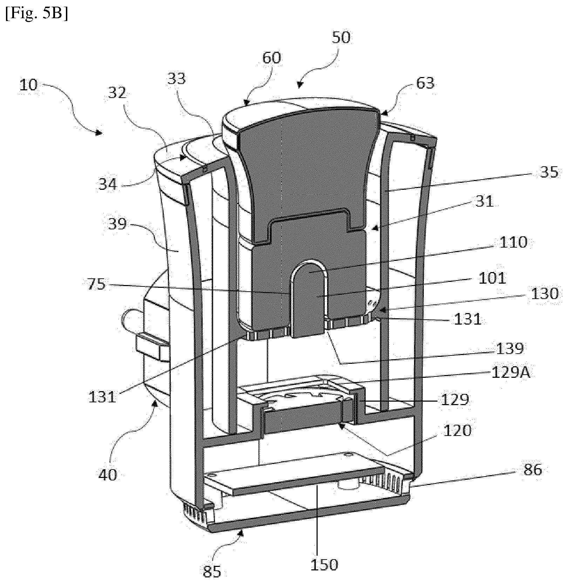

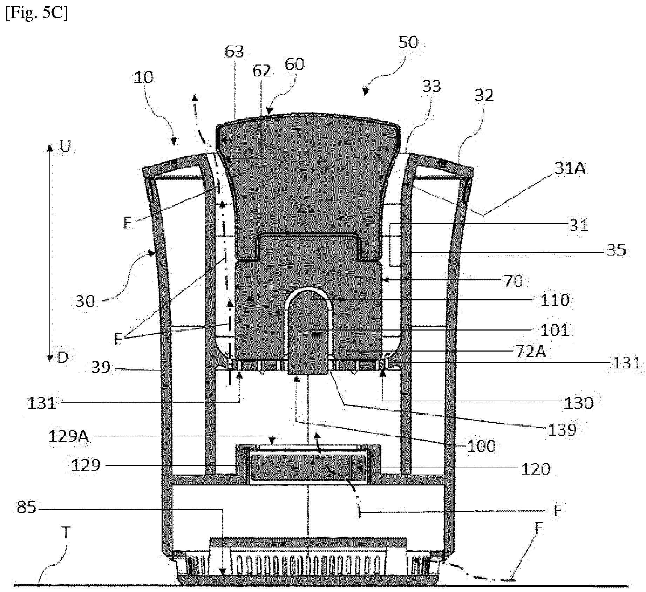

On décrit tout d'abord la partie fixe 20. La partie fixe 20 comprend un boîtier 30 et une prise d'alimentation électrique 40. Les figures 5A, 5B et 5C sont des vues en coupe de l'appareil 10 selon le plan V-V de la [Fig.2] et révèlent ainsi des composants de l'appareil 10 disposés à l'intérieur du boîtier 30.

Le boîtier 30 définit un réceptacle 31. Le réceptacle 31 est destiné à recevoir un ensemble amovible 50 décrit ci-après. Le réceptacle 31 présente ici une section en forme de carré arrondi, mais il pourrait également s'agir d'une section circulaire, elliptique ou même polygonale. Le réceptacle 31 débouche sur une paroi supérieure 32 du boîtier 30 par une ouverture 33. Le réceptacle 31 est défini par une paroi interne 35 (cf. [Fig.5A], [Fig.5B] et [Fig.5C]) du boîtier 30 qui s'étend depuis la paroi supérieure 32 vers l'intérieur du boîtier 30. La référence 39 sur les figures désigne une paroi latérale extérieure du boîtier 30.

La prise d'alimentation électrique 40 est rendue solidaire du boîtier 30 de toute façon appropriée. Dans l'exemple représenté, la prise d'alimentation électrique 40 est une prise d'alimentation secteur. L'appareil 10 peut ainsi être alimenté en électricité sur secteur, ce qui permet notamment une utilisation dans un lieu clos tel qu'une serre ou un bâtiment, par exemple, ou dans un lieu à l'abri des précipitations. Toutefois, d'autres types de prises d'alimentation électrique 40 sont envisageables, notamment des prises d'alimentation électrique en courant continu.

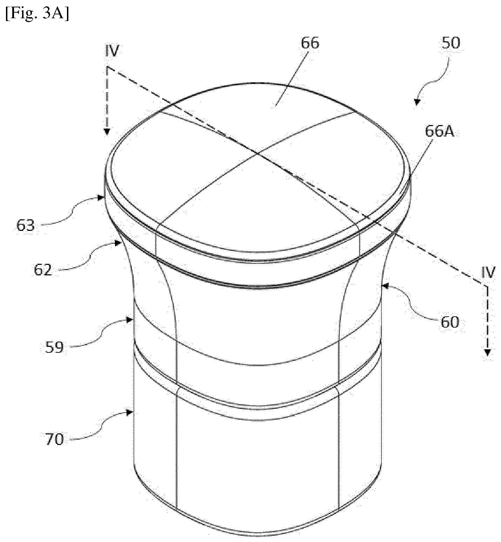

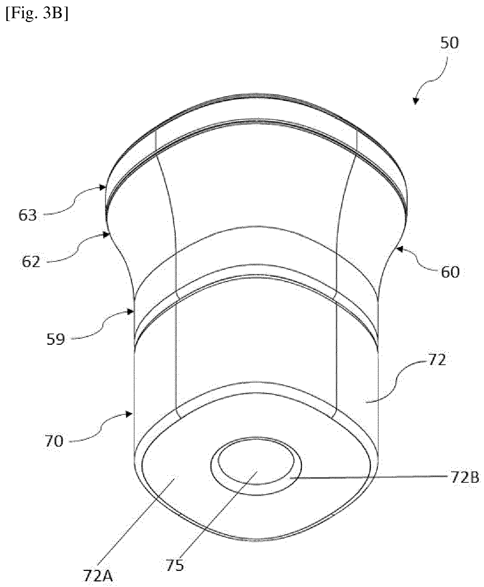

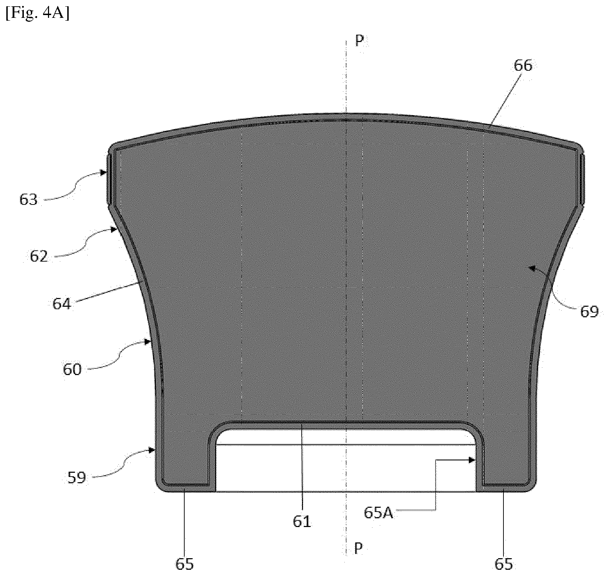

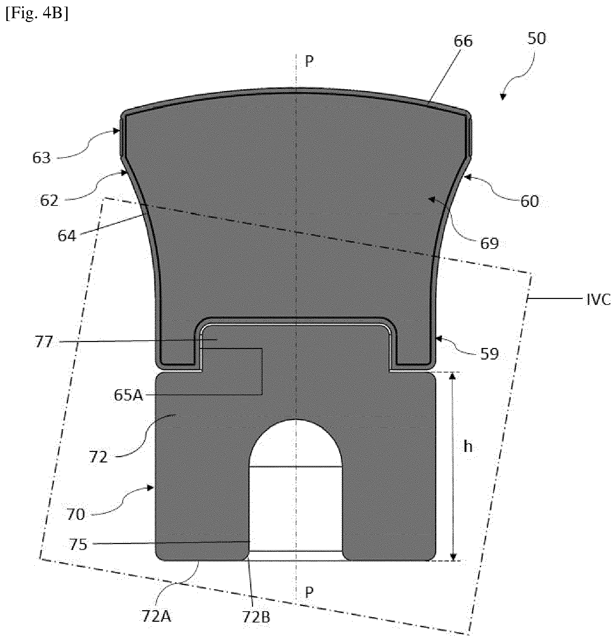

On décrit maintenant l'ensemble amovible 50 en se référant aux figures 3A, 3B, 4A et 4B. L'ensemble amovible 50 comprend un récipient de stockage 60 et une mèche 70. La mèche 70 est solidaire du récipient de stockage 60 de sorte que l'ensemble amovible 50 peut être inséré d'un seul tenant dans le réceptacle 31 en saisissant l'ensemble amovible 50.

La [Fig.4A] est une vue en coupe du récipient de stockage 60 seul. Comme cela est mieux visible sur la [Fig.4A], une portion inférieure 59 du récipient de stockage 60 présente une section de cylindre droit dont la directrice est un axe principal P-P et dont la génératrice est ici en forme de carré arrondi. Dit autrement, une section du récipient de stockage 60, perpendiculairement à l'axe P-P, au niveau de la portion inférieure 59, est en forme de carré arrondi. Cette section est de préférence identique à la section du réceptacle 31. Le volume intérieur 69 du récipient de stockage 60 est délimité par une paroi périphérique 64, par une paroi supérieure 66, et par une paroi inférieure 65.

Comme cela est mieux visible sur la [Fig.4A], la paroi inférieure 65 comporte un lamage 65A. Un orifice de vidange 61 est pratiqué dans tout ou partie du fond du lamage 65A. L'orifice de vidange 61 est orienté vers le bas dans une position d'utilisation de l'ensemble amovible 50, cette position d'utilisation étant représentée sur la [Fig.2], la [Fig.5A], la [Fig.5B] et la [Fig.5C].

On précise que les termes « vers le bas », « inférieur » et « supérieur » s'entendent par rapport à l'accélération de la pesanteur dans la position d'utilisation.

De même, les termes « en dessous » et « au-dessus » s'entendent par rapport à l'accélération de la pesanteur dans la position d'utilisation.

De façon équivalente, on peut attribuer à l'appareil 10 une direction descendante D et une direction montante U (cf. [Fig.5A] et [Fig.5C]), la direction descendante D et la direction montante U étant opposées. Dans la position d'utilisation, par rapport à l'accélération de la pesanteur, la direction descendante D est vers le bas et la direction montante U est vers le haut ; plus précisément, la direction descendante D est parallèle à l'accélération de la pesanteur et orientée vers le sol, et la direction montante U est opposée à la direction descendante D. Les termes « vers le bas », « inférieur » ou « en-dessous » s'entendent alors suivant la direction descendante D ; les termes « supérieur » et « au-dessus » s'entendent alors suivant la direction montante U.

Dans la position d'utilisation, l'axe P-P peut être parallèle à l'accélération de la pesanteur et donc à la direction descendante D et à la direction montante U.

Le récipient de stockage 60 peut présenter une symétrie de révolution autour de l'axe P-P.

Dans un exemple de réalisation, le récipient de stockage 60 est réalisé par moulage par injection d'un matériau plastique adapté, tel que du polypropylène (PP) par exemple. Un ou plusieurs évents de pressurisation (non représentés) peuvent être pratiqués dans la paroi supérieure 66 et/ou la paroi périphérique 64 afin de garantir que la substance liquide contenue dans le récipient de stockage 60 peut continuer à s'écouler à mesure que le récipient de stockage 60 est vidé. En variante, le récipient de stockage 60 ne présente pas d'autre ouverture que l'orifice de vidange 61, et contient, outre la substance liquide, une phase gazeuse occupant au moins 20% du volume du récipient de stockage 60.

Une portion intermédiaire 62 du récipient de stockage 60 est divergente, c'est-à-dire conformée de telle sorte que la paroi périphérique 64 s'éloigne de l'axe principal P-P en s'éloignant de la portion inférieure 59. Cette portion intermédiaire 62 est prolongée par une portion terminale 63 au niveau de laquelle la paroi périphérique 64 est de nouveau parallèle à l'axe principal P-P. On comprend que la portion intermédiaire 62 et la portion terminale 63 facilitent la préhension du récipient de stockage 60 par un utilisateur, puisque celui-ci peut aisément saisir le récipient de stockage 60 en amenant ses doigts autour de la portion terminale 63 puis en plaçant l'extrémité de ses doigts sur la portion intermédiaire 62.

Le volume intérieur 69 du récipient de stockage 60 peut optionnellement être au moins en partie rempli d'une mousse polymère (non représentée) imprégnée de la substance liquide. En alternative, le volume intérieur 69 peut simplement être rempli de la substance liquide.

Selon certaines variantes non représentées, le volume intérieur 69 peut contenir plusieurs substances liquides différentes. Pour cela le volume intérieur 69 peut être cloisonné de façon à définir plusieurs sous-volumes intérieurs, chaque volume intérieur 69 contenant une substance liquide et présentant un orifice de vidange 61 pour cette substance liquide. Selon encore d'autres variantes non représentées, l'ensemble amovible 50 peut comprendre plusieurs récipients de stockage 60, éventuellement identiques, contenant chacun une substance liquide et tous reliés à la mèche 70 qui va maintenant être décrite. Ces variantes permettent de fournir un ensemble amovible 50 contenant plusieurs substances liquides qui ne se mélangent qu'au moment de leur diffusion par l'appareil 10.

Il est à noter que la paroi supérieure 66 peut éventuellement être matérialisée par un couvercle qui est amovible de façon à permettre de remplir de nouveau le volume intérieur 69 du récipient de stockage 60 lorsque celui-ci a été vidé de substance liquide. La référence 66A sur la [Fig.3A] désigne un chanfrein qui peut permettre de faciliter le retrait d'un tel couvercle.

On a représenté en coupe sur la [Fig.4B] le récipient de stockage 60 ensemble avec une mèche 70.

La mèche 70 est positionnée en sortie de l'orifice de vidange 61, de sorte que la substance liquide peut passer de l'espace intérieur 69 du récipient de stockage 60 à la mèche 70 et par là à des surfaces d'évaporation constituées par les parois extérieures de la mèche 70. La mèche 70 comporte ici une portion principale 72 de même section que la portion inférieure 59 du récipient de stockage 60. En outre, un évidement 75 s'étend dans la mèche 70, ici parallèlement à l'axe P-P, depuis une surface inférieure 72A de la portion principale 72. L'évidement 75 est de préférence aveugle comme représenté sur les figures.

La mèche 70 peut présenter une symétrie de révolution autour de l'axe P-P.

La mèche 70 peut être rapportée au récipient de stockage 60 de diverses manières. Dans l'exemple représenté sur les figures, la mèche 70 comporte un ergot 77 destiné à être reçu dans le lamage 65A, par exemple enfoncé en force dans le lamage 65A.

La mèche 70 est réalisée partiellement ou entièrement en un matériau poreux, par exemple en bois, en textile, en céramique ou en polymère. Un autre exemple de matériau poreux possible est un matériau métallique poreux obtenu par frittage d'une poudre métallique ou d'une poudre d'alliage métallique. De tels matériaux métalliques poreux sont connus en tant que tels et les techniques permettant de les obtenir ne sont pas décrites en détail ici.

Comme on l'a déjà mentionné ci-dessus, le récipient de stockage 60 comporte un orifice de vidange 61 qui est orienté vers le bas dans une position d'utilisation de l'ensemble amovible 50. La mèche 70 est positionnée en sortie de cet orifice de vidange 61 de façon à recevoir et être imprégnée, du fait de sa porosité, de la substance liquide contenue dans le volume intérieur 69 du récipient de stockage 60.

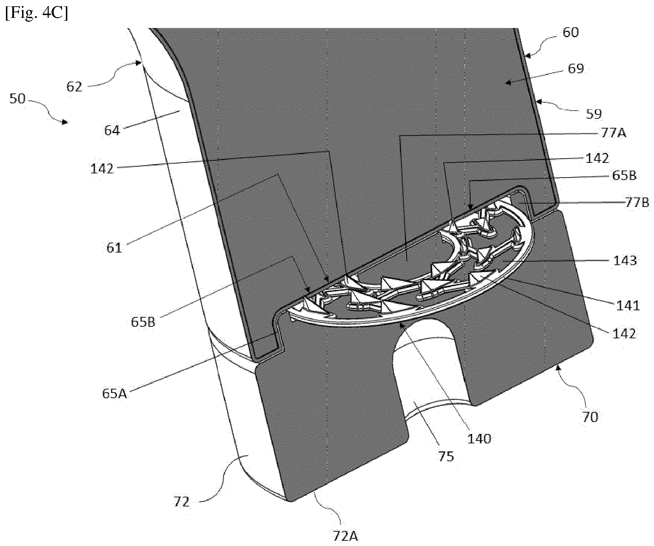

La [Fig.4C] est une vue agrandie du détail IVC de la [Fig.4B] et montre ainsi la liaison entre la mèche 70 et l'orifice de vidange 61 dans un exemple de réalisation particulier de l'ensemble amovible 50. Dans l'exemple de réalisation représenté, l'orifice de vidange 61 est obturé par un opercule 65B. La mèche 70 présente un ergot central 77A. L'ensemble amovible 50 comporte en outre un dispositif de perforation 140 disposé entre la mèche 70 et l'orifice de vidange 61. Le dispositif de perforation 140 comporte un corps principal 141 annulaire emmanché sur l'ergot central 77A de la mèche 70, et optionnellement maintenu en position par rapport à l'ergot central 77A par une saillie annulaire 77B que présente la mèche 70 autour de l'ergot central 77A. Le corps principal 141 présente une pluralité de dents 142, et, entre les dents 142, une pluralité de lumières 143. Les dents 142 sont disposées de façon à pouvoir perforer l'opercule 65B. Une fois que l'opercule 65B est perforé par les dents 142, la substance liquide s'écoule à travers les trous réalisés par les dents 142, puis à travers les lumières 143, et vient mouiller l'ergot 77, puis le reste de la mèche 70 par capillarité. Cette perforation de l'opercule 65B par les dents 142 peut notamment être obtenue en déplaçant la mèche 70 en direction de l'orifice de vidange 61. Le réceptacle 31 et la mèche 70 peuvent être conformés de telle sorte que ce déplacement de la mèche 70 en direction de l'orifice de vidange 61 se produit lors de l'installation de l'ensemble amovible 50 dans le réceptacle 31.

Le dispositif de perforation 140 peut être par exemple réalisé par moulage par injection d'un polymère approprié. En variante, il pourrait aussi s'agir d'une pièce métallique, par exemple moulée, ou encore d'une pièce en céramique. En outre, le dispositif de perforation 140 pourrait être constitué de plusieurs secteurs annulaires, éventuellement mais non obligatoirement solidaires les uns des autres.

Les dents 142 et les lumières 143 sont de préférence régulièrement espacées de façon à favoriser une imprégnation uniforme de la mèche 70 par la substance liquide.

La géométrie des dents 142 et des lumières 143 représentée sur la [Fig.4C] n'est qu'un exemple. De nombreuses autres configurations sont possibles. En outre, en complément ou en remplacement des dents 142, des aiguilles, creuses ou non, peuvent être disposées sur le dispositif de perforation 140 de façon à perforer l'opercule 65B comme on vient de le décrire.

La liaison qui vient d'être décrite entre la mèche 70 et le volume intérieur 69 du récipient de stockage 60 via l'orifice de vidange 61 n'est qu'un exemple. De nombreuses autres liaisons sont possibles. Notamment, lorsque le volume intérieur 69 du récipient de stockage 60 est au moins en partie rempli d'une mousse polymère imprégnée de la substance liquide, la mèche 70 peut présenter un ou plusieurs ergots qui traversent l'orifice de vidange 61 jusqu'à venir au contact de cette mousse polymère. Un ou plusieurs joints d'étanchéité (non représentés) peuvent alors être disposés de façon à garantir une liaison étanche entre la mèche 70 et l'orifice de vidange 61.

Selon encore une autre variante non représentée, l'ergot 77 peut être configuré de telle sorte que la mèche 70 peut être vissée dans l'orifice de vidange 61 du récipient de stockage 60. Optionnellement dans ce cas, l'ergot 77 peut comporter des microaiguilles (non représentées) venant perforer l'opercule 65B pendant ce vissage.

Comme on l'a mentionné ci-dessus, l'ensemble amovible 50 est destiné à être reçu dans le réceptacle 31 défini par le boîtier 30. La [Fig.2], la [Fig.5A], la [Fig.5B] et la [Fig.5C] représentent l'ensemble amovible 50 en position dans le réceptacle 31 dans la position d'utilisation. Comme représenté sur ces figures, dans la position d'utilisation, la portion terminale 63 est en saillie par rapport à la paroi supérieure 32 du boîtier 30. De façon équivalente, la portion terminale 63 disposée est au-dessus de l'ouverture 33, c'est-à-dire au-delà de l'ouverture 33 suivant la direction montante U. Ceci facilite la préhension du récipient de stockage 60 et le remplacement de l'ensemble amovible 50. Bien que cela ne soit pas représenté sur les dessins, la portion terminale 63 pourrait même présenter une plus grande dimension extérieure approximativement égale à, ou même supérieure à, la plus grande dimension de l'ouverture 33 du réceptacle 31. En variante, la portion terminale 63 peut affleurer la paroi supérieure 32 ou être en-dessous de la paroi supérieure 32, c'est-à-dire au-delà de la paroi supérieure 32 suivant la direction descendante D.

En revenant à la [Fig.1], on visualise le fond du réceptacle 31 en vue de dessus depuis le dessus de l'appareil 10. Un organe de chauffage, généralement désigné par la référence 100 et partiellement représenté sur les vues en coupe des figures 5A, 5B et 5C, est disposé dans le boîtier de diffusion 30. L'organe de chauffage 100 comporte une tige 101 portant à son extrémité supérieure une résistance électrique 110. La tige 101 peut s'étendre à travers l'évidement 75, de telle sorte que la résistance électrique 110 vient au contact des parois de l'évidement 75 lorsque l'ensemble amovible 50 est en place dans le réceptacle 31. En alimentant ou non en électricité la résistance électrique 110, on peut mettre en œuvre un contrôle de l'écoulement de la substance liquide à travers la mèche 70 selon les principes physiques décrits dans les documents WO 2019/243734 A1 et WO 2020/254733 A1.

Optionnellement, la surface inférieure 72A de la mèche 70 peut être reliée à l'évidement 75 par un pan incliné 72B (cf. [Fig.3B] et [Fig.4B]), de façon à faciliter l'insertion de la tige 101 et de la résistance électrique 110 dans l'évidement 75.

En variante, la mèche 70 peut ne pas présenter d'évidement 75 ; la résistance électrique 110 est alors placée directement sur une surface extérieure de la mèche 70, par exemple sur la surface inférieure 72A.

L'organe de chauffage 100 peut comprendre une carte électronique 150 (représentée schématiquement sur la [Fig.5A], la [Fig.5B] et la [Fig.5C]) pour l'alimentation électrique et la commande de la résistance électrique 110. Par exemple un dispositif de commande (non représenté), tel qu'un microprocesseur, peut être agencé sur la carte électronique 150 afin de commander la résistance électrique 110 en fonction d'une température de consigne de la mèche 70. Cette température de consigne peut être relevée par un capteur de température (non représenté), porté par exemple par la tige 101. La carte électronique 150 est ici portée par la pièce de couvercle 85 décrite plus loin, mais peut en variante être disposée à divers emplacements dans le boîtier 30.

L'appareil 10 comporte en outre un système d'aération. Ce système d'aération comporte une entrée d'air 98 et un ventilateur 120. L'entrée d'air 98 est disposée en-dessous de l'ensemble amovible 50 dans la position d'utilisation. Le ventilateur 120, lequel est visible sur les vues en coupe des figures 5A, 5B et 5C, est disposé en-dessous de l'ensemble amovible 50 dans la position d'utilisation. On comprend que de cette manière, le ventilateur 120 amène un débit d'air depuis l'entrée d'air 98 vers les surfaces d'évaporation constituées par les parois extérieures de la mèche 70, puis vers l'ouverture 33. Les flèches F en trait mixte sur la [Fig.5A] indiquent le sens et la direction de ce débit d'air. Ainsi, lorsque le ventilateur 120 et la résistance électrique 110 fonctionnent, l'appareil 10 génère un débit d'air chargé en substance évaporée, ce débit d'air sortant par l'ouverture 33. En d'autres termes, l'ouverture 33 forme la sortie d'air unique du système d'aération de l'appareil 10.

En se référant plus particulièrement à la [Fig.5A], on constate que, en regard de la portion intermédiaire 62 du récipient de stockage 60, le réceptacle 31 peut présenter une portion divergente 31A, au niveau de laquelle les parois du réceptacle 31 sont courbées de façon à élargir la section du réceptacle 31. Par exemple les parois du réceptacle 31 au niveau de la portion divergente 31A peuvent présenter une courbure identique ou sensiblement identique à la courbure de la portion intermédiaire 62. Les courbures de la portion divergente 31A et de la portion intermédiaire 62 tendent à dévier le débit d'air au voisinage de l'ouverture 33, comme représenté par les flèches F. Ceci peut tendre à davantage disperser l'air chargé en substance évaporée dans l'air ambiant.

L'entrée d'air 98 peut être réalisée de diverses manières. Dans l'exemple représenté, l'extrémité inférieure du boîtier 30 est fermée par une pièce de couvercle 85 (cf. figures 2, 5A, 5B et 5C). La pièce de couvercle 85 présente une pluralité d'ouvertures 86 matérialisant l'entrée d'air 98. Les ouvertures 86 peuvent ou non prolonger des ouvertures correspondantes (non représentées) que présente le boîtier 30 au droit de la pièce de couvercle 85. La pièce de couvercle 85 peut être réalisée d'une seule pièce avec le boîtier 30 ou bien fixée au boîtier 30. En variante, la pièce de couvercle 85 peut être fixée au boîtier 30 de façon amovible. Notamment dans ce cas, l'extrémité inférieure du boîtier 30 peut être reliée à une canalisation d'air ou une autre arrivée d'air ; et le ventilateur 120 peut être déporté dans cette canalisation ou arrivée d'air.

Dans l'exemple représenté, l'extrémité inférieure du réceptacle 31 est fermée par une pièce de guidage d'air 130 (cf. [Fig.1], [Fig.5A], [Fig.5B] et [Fig.5C]) qui est une paroi de fond protégeant notamment le ventilateur 120 et les composants électriques d'un accès par l'utilisateur. La pièce de guidage d'air 130 comporte ici une pluralité de trous traversants 131 qui laissent passer le débit d'air créé par le ventilateur 120. La pièce de guidage d'air 130 présente en outre un orifice central traversant 139 pour laisser passer la tige 101 et la résistance chauffante 110. La tige 101 peut éventuellement être solidaire de la pièce de guidage d'air 130.

La pièce de guidage d'air 130 est ici évasée, mais en variante, elle pourrait être plane. On précise en outre que bien que les dessins représentent la surface inférieure 72A de la mèche 70 comme étant quasiment au contact de la pièce de guidage d'air 130, un certain espace, de l'ordre de quelques millimètres ou quelques centimètres, par exemple, peut être ménagé entre la surface inférieure 72A et la pièce de guidage d'air 130 de façon à permettre une évaporation de la substance liquide au niveau de la surface inférieure 72A.

Le nombre, la disposition et les orientations relatives des trous traversants 131 peuvent être choisis de diverses façons pour orienter le débit d'air sur les parois extérieures de la mèche 70 et par là favoriser l'évaporation de la substance sur les parois extérieures de la mèche 70. En outre, bien que les figures montrent que la pièce de guidage d'air 130 comporte un grand nombre de trous traversants 131 de section circulaire ou ovoïde de faibles dimensions (diamètre intérieur de l'ordre de 0,1 mm à 4 mm, par exemple), d'autres dimensions et formes de trous traversants sont possibles. Par exemple, dans une variante non représentée, la pièce de guidage d'air 130 comporte, en complément ou en remplacement des trous traversants 131, un orifice traversant annulaire ou circulaire. Dans cette variante, la pièce de guidage d'air 130 peut éventuellement porter la tige 101 ; l'orifice traversant annulaire ou circulaire peut être disposé sous la tige 101.

Diverses constructions peuvent être retenues pour le ventilateur 120. Dans l'exemple représenté, le ventilateur 120 est un ventilateur axial. Le ventilateur 120 est ici maintenu en place dans un renfoncement interne 129 que présente le boîtier 30 sous l'organe de chauffage 100 et la pièce de guidage d'air 130. Le fond de ce renfoncement interne 129 présente un orifice traversant 129A disposé entre la mèche 70 et le ventilateur 120 pour laisser passer le débit d'air. En variante, le ventilateur 120 peut être maintenu en place différemment dans le boîtier 30. En outre, en variante, le ventilateur 120 peut être un ventilateur centrifuge ou encore d'autres types de ventilateur.

Un filtre à air (non représenté) peut éventuellement être disposé entre l'entrée d'air 98 et le ventilateur 120 afin de limiter le risque d'encrassement de la mèche 70 par des particules extérieures indésirables.

Bien que les figures montrent que l'entrée d'air 98 est disposée à l'extrémité inférieure du boîtier 30, en-dessous du ventilateur 120, qui est lui-même disposé en-dessous de la mèche 70, de nombreux autres positionnements de l'entrée d'air 98 et du ventilateur 120 dans le boîtier 30 sont possibles.

Notamment, l'entrée d'air 98 peut être disposée sur la paroi latérale extérieure 39 du boîtier 30. Dans ce cas, l'entrée d'air 98 est éventuellement, mais non obligatoirement, plus loin vers le bas de la paroi supérieure 32 que la mèche 70, c'est-à-dire au-delà de la mèche 70 suivant la direction descendante D. Le ventilateur 120 peut être disposé en-dessous de la mèche 70 comme représenté sur les figures, ou bien être disposé entre la paroi latérale extérieure 39 et la paroi interne 35 définissant le réceptacle 31. Bien entendu, dans ce dernier cas, il est possible que des trous traversants analogues aux trous traversants 131 de la pièce de guidage d'air 130 soient pratiqués dans la paroi interne 35, en regard de la mèche 70. Dans ce dernier cas également, le ventilateur 120 est éventuellement, mais non obligatoirement, plus loin vers le bas de la paroi supérieure 32 que la mèche 70, c'est-à-dire au-delà de la mèche 70 suivant la direction descendante D.

Le ventilateur 120 peut être commandé par le dispositif de commande mentionné ci-dessus.

L'ensemble amovible 50 peut être maintenu en place dans le réceptacle 31 de diverses manières, tant que l'ensemble amovible 50 peut être retiré du réceptacle 31 afin d'être remplacé à l'identique lorsque la substance liquide contenue dans le récipient de stockage 60 est épuisée. Par exemple l'ensemble amovible 50 peut être vissé ou encliqueté dans le réceptacle 31. Dans un mode de réalisation, l'ensemble amovible 50 se limite au récipient 60, tandis que la mèche 70 reste à demeure dans l'appareil 10. Dans un autre mode de réalisation, la mèche 70 peut être démontée et remplacée indépendamment du récipient 60.

Le boîtier 30 peut être muni d'un témoin lumineux 34, indiquant par exemple l'état de fonctionnement de l'appareil 10 en fonction de commandes fournies par le dispositif de commande mentionné ci-dessus. Dans l'exemple représenté sur les figures, ce témoin lumineux 34 prend la forme d'un anneau translucide éclairé par une ou plusieurs diodes électroluminescentes de couleur et s'étendant sur la paroi supérieure 32 du boîtier autour de l'ouverture 33. Toutefois, de nombreuses autres mises en œuvre du témoin lumineux 34 sont possibles.

La [Fig.5C] montre qu'une façon possible d'utiliser l'appareil 10 consiste à le poser par son extrémité inférieure sur une surface plane et horizontale T. On précise qu'une surface est « horizontale » lorsqu'elle est perpendiculaire à l'accélération de la pesanteur. La surface T peut être la surface d'une table ou plus généralement d'un meuble. Pour permettre de poser l'appareil 10 par son extrémité inférieure sur la surface T, la surface inférieure de la pièce de couvercle 85 est plane comme représenté sur les figures.

### Exemples

Deux appareils diffuseurs 10 décrits ci-dessus ont été fabriqués, à l'identique excepté pour la construction de leur mèche 70. Les deux mèches 70 différentes des deux appareils diffuseurs 10, ci-après dénommés respectivement « système 1 » et « système 2 », présentent les caractéristiques ci-dessous :
- système 1 : la mèche 70 est réalisée en alumine frittée, et présente une taille de pores de 120 nm et une porosité uniforme de 40%. La hauteur h (cf. [Fig.4B]) de la mèche 70 est de 8 mm ;
- système 2 : la mèche 70 est réalisée en alumine frittée, et présente une taille de pores de 120 nm et une porosité uniforme de 40%. La hauteur h (cf. [Fig.4B]) de la mèche 70 est de 25 mm.

Trois expériences, ci-après dénommées « expérience 1 », « expérience 2 » et « expérience 3 », ont été réalisées avec le système 1 ou le système 2.

Dans ces trois expériences, on mesure un taux de diffusion, aussi appelé taux d'évaporation. Les mesures de ces taux sont réalisées selon un protocole de mesure gravimétrique qui consiste à mesurer la perte de masse relative du système au cours du temps en considérant (par constat visuel) que toute perte de masse relève d'une évaporation puis expulsion des molécules hors du système, à l'exclusion de toute fuite de liquide.

Certaines des huiles essentielles ou fragrances alimentaires utilisées dans les expériences ci-après ont été préalablement diluées dans du DPG (dipropylène glycol ; substance présentant à pression atmosphérique une température de fusion de -39°C et une température d'ébullition de 230°C. La dilution dans du DPG est exprimée en pourcentage massique, avec la convention ci-après : un mélange 25% huile essentielle de rhubarbe-DPG contient 25% en masse d'huile essentielle rhubarbe dans du DPG. Un mode de préparation d'un tel mélange consiste donc à ajouter 75 g de DPG à 25 g d'huile essentielle de rhubarbe.

### Expérience 1

Dispositif expérimental : Trois substances différentes ont été successivement diffusées avec le système 1 : huile essentielle de rhubarbe, huile essentielle de pin eucalyptus, huile essentielle de rose, le récipient de stockage 60 étant rempli à chaque fois avec 5 g d'huile essentielle, celle-ci n'étant pas diluée par ajout d'un solvant. On a laissé à chaque fois fonctionner le système 1 à une température ambiante de 20°C, en maintenant la mèche 70 à une température de 35°C, et en faisant fonctionner le ventilateur 120 de façon à créer un débit d'air constant égal à 4 m³/h.

Résultat : Les trois substances testées s'écoulent à travers la mèche 70 et s'évaporent, sans aucune recondensation observée, avec des taux de diffusion variables. La perte en poids est linéaire au cours du temps, contrairement à ce que l'on observe avec des diffuseurs passifs pour lesquels le taux de diffusion est fonction de la concentration. Le ressenti olfactif est extrêmement puissant. Les taux de diffusion mesurés avec chaque substance sont consignés dans le tableau 1 ci-dessous.

**[Tableaux1]**

| Substance | Huile essentielle de rhubarbe non diluée | Huile essentielle de pin eucalyptus non diluée | Huile essentielle de rose non diluée |
|---|---|---|---|
| Taux de diffusion en gramme/jour | 0,8 | 1,6 | 0,9 |

### Expérience 2

Dispositif expérimental : Trois substances différentes ont été successivement diffusées avec le système 1 : huile essentielle de pin eucalyptus non diluée, mélange 25% huile essentielle de pin eucalyptus-DPG, mélange 50% huile essentielle de pin eucalyptus-DPG, le récipient de stockage 60 étant rempli à chaque fois de 5 g en masse totale de substance. On a laissé à chaque fois fonctionner le système 1 à une température ambiante de 20°C, en maintenant la mèche 70 à une température de 35°C, et en faisant fonctionner le ventilateur 120 de façon à créer un débit d'air constant égal à 4 m³/h.

Résultat : Les substances testées s'écoulent à travers la mèche 70 et s'évaporent avec des taux de diffusion différents, qui sont consignés dans le tableau 2 ci-dessous. Aucune recondensation n'a été observée. La perte en poids est linéaire au cours du temps, contrairement à ce que l'on observe avec des diffuseurs passifs pour lesquels le taux de diffusion est fonction de la concentration. Il a été constaté qualitativement que le rendu olfactif est moins puissant avec une dilution plus importante avec du DPG. Il a été constaté qualitativement que les substances parfumaient une pièce de 120 m² en moins de 15 minutes.

**[Tableaux2]**

| Substance | Huile essentielle de pin eucalyptus non diluée | Mélange 25% huile essentielle de pin eucalyptus-DPG | Mélange 50% huile essentielle de pin eucalyptus-DPG |
|---|---|---|---|
| Taux de diffusion en gramme/jour | 1,66 | 1,0 | 0,5 |

### Expérience 3

Dispositif expérimental : Cinq substances ont été successivement diffusées avec le système 2 : mélange 10% huile essentielle odeur marine-DPG, mélange 50% huile essentielle odeur marine-DPG, huile essentielle odeur marine non diluée, mélange 50% fragrance alimentaire pièce montée-DPG, mélange 2% fragrance alimentaire homard-DPG, le récipient de stockage étant rempli à chaque fois de 15 g en masse totale de substance. On a laissé à chaque fois fonctionner le système 2 à une température ambiante de 20°C, en maintenant la mèche 70 à une température de 32°C, et en faisant fonctionner le ventilateur 120 de façon à créer un débit d'air constant égal à 4 m³/h. Les taux de diffusion mesurés avec chaque fragrance alimentaire sont consignés dans le tableau 1 ci-dessous.

Résultat : Les substances testées s'écoulent à travers la mèche 70 et s'évaporent avec des taux de diffusion différents, qui sont consignés dans le tableau 3 ci-dessous (on précise que dans le tableau 3, « h.e. » signifie en abrégé huile essentielle et « f.a. » signifie en abrégé fragrance alimentaire). La perte en poids est linéaire au cours du temps. La qualité du ressenti olfactif est excellente et constante dans le temps.

**[Tableaux3]**

| Substance | Mélange 10% h.e. marine-DPG | Mélange 50% h.e. marine-DPG | h.e. marine non diluée | Mélange 50% f.a. pièce montée-DPG | Mélange 2% f.a. homard-DP G |
|---|---|---|---|---|---|
| Taux de diffusion en mg/heure | 6,2 | 5,5 | 5,2 | 4,0 | 29,2 |

### Variantes de réalisation

La construction de l'appareil 10 représentée sur les figures 1 à 5C n'est qu'un exemple. De nombreuses autres constructions sont envisageables.

Selon des variantes non représentées, l'ouverture 33 peut former l'une des sorties d'air du système d'aération de l'appareil 10, une ou plusieurs autres sorties d'air supplémentaires étant formées dans le boîtier 30 et étant en communication fluide avec le réceptacle 31. Dans ce cas, une partie de l'air chargé en substance évaporée est amené par le système d'aération vers ces sorties d'air supplémentaires au lieu d'être amené vers l'ouverture 33.

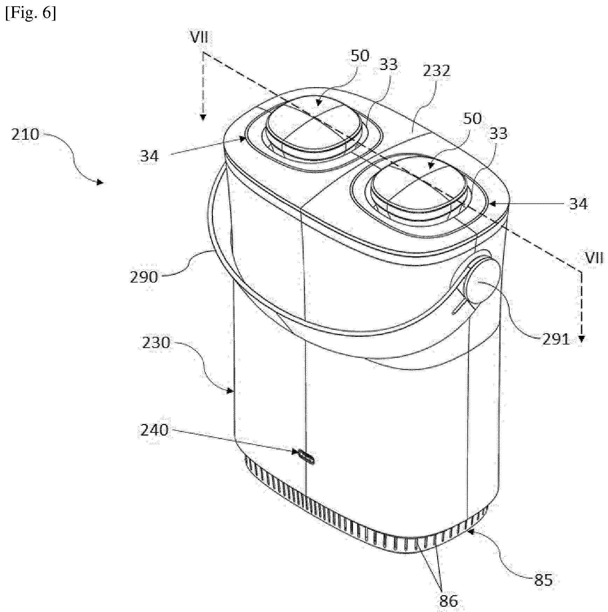

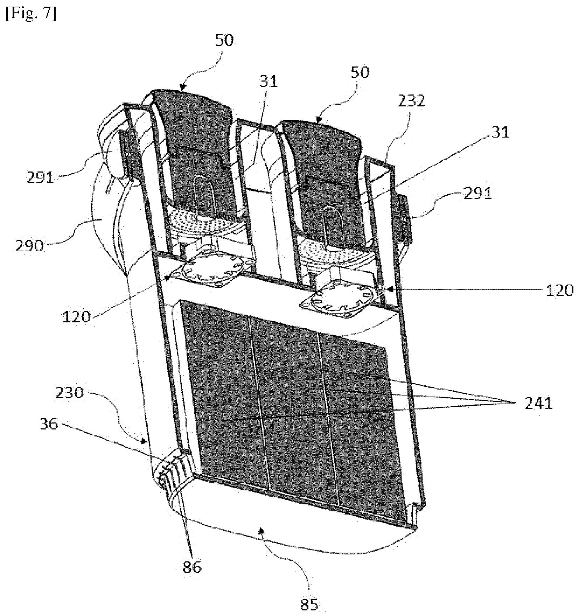

Une deuxième variante de réalisation d'un appareil 210 est représentée en perspective sur la [Fig.6] et en coupe sur la [Fig.7]. Sur ces figures, les éléments identiques à ceux décrits plus haut en rapport avec les figures 1 à 5C portent des références identiques et ne sont pas décrits en détail à nouveau. L'appareil 210 se distingue de l'appareil 10 en cela que le boîtier 230 présente deux réceptacles 31 dont les ouvertures 33 débouchent toutes les deux sur la paroi supérieure 232 du boîtier 230. Chacun des deux réceptacles 31 reçoit un ensemble amovible 50. L'appareil 210 peut ainsi être utilisé pour diffuser deux substances différentes, en insérant dans chacun des deux réceptacles 31 deux ensembles amovibles 50 contenant des substances différentes. Bien entendu, un nombre plus important de réceptacles 31 et d'ensembles amovibles 50 peut être prévu dans le même appareil 210 si cela est souhaité. On prévoit ici qu'à chacun des réceptacles 31 est associé un ventilateur 120, mais en variante, un seul ventilateur 120 peut être associé à tous les réceptacles 31.

On voit également sur la [Fig.6] que l'appareil 210 est alimenté en électricité par une prise d'alimentation en courant continu 240, ici du type USB-C, et on voit sur la [Fig.7] que l'appareil 210 comprend plusieurs batteries 241, par exemple du type lithium-ion, disposées à l'intérieur du boîtier 230. Ainsi, l'appareil 210 peut fonctionner même en l'absence d'alimentation secteur.

En outre, on prévoit que l'appareil 210 est pourvu d'une anse de transport 290, ici articulée à deux extrémités du boîtier 230 par deux articulations opposées 291. Ainsi, l'appareil 210 peut aisément être transporté à la main par un utilisateur.

Toutefois, en variante, l'appareil 210 peut être alimenté sur secteur comme l'appareil 10 et/ou être dépourvu de l'anse de transport 290.

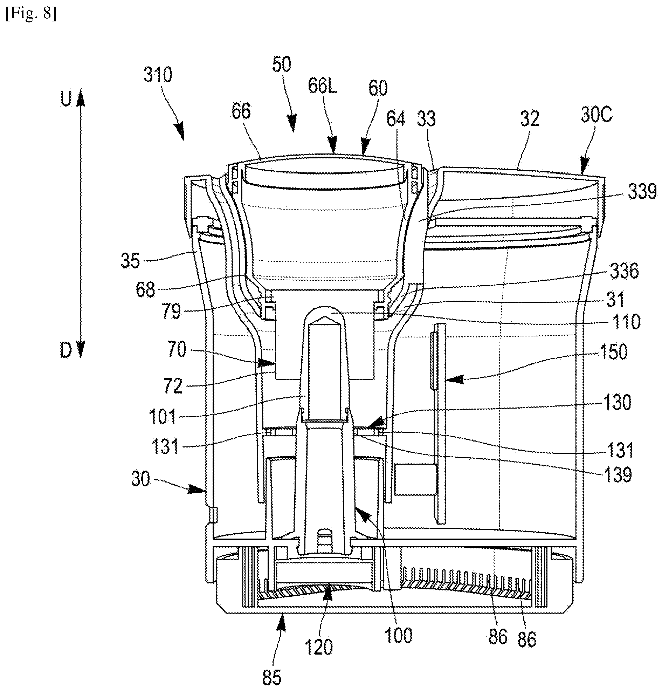

Une troisième variante de réalisation d'un appareil 310 est représentée en coupe sur la [Fig.8]. Sur cette figure, les éléments identiques à ceux décrits plus haut en rapport avec les figures 1 à 5C portent des références identiques et ne sont pas décrits en détail à nouveau.

L'appareil 310 se distingue de l'appareil 10 en cela que le réceptacle 31 comporte une paroi de séparation interne 336. La paroi de séparation interne 336 est disposée entre la paroi périphérique 64 du récipient de stockage 60 et la paroi interne 35 définissant le réceptacle 31.

La mèche 70, ou à tout le moins une partie inférieure de sa portion principale 72, n'est pas entourée par la paroi de séparation interne 336, tandis que la paroi périphérique 64 du récipient de stockage 60 est entourée entièrement, par la paroi de séparation interne 336. Ainsi, le débit d'air chargé en substance évaporée au niveau de la mèche 70 ne circule pas le long de la paroi périphérique 64, mais le long de la paroi de séparation interne 336. Par conséquent, s'il se produit une recondensation de la substance évaporée dans le réceptacle 31, cette recondensation ne se produit pas sur la paroi périphérique 64, mais sur la paroi de séparation interne 336. Le risque qu'un utilisateur de l'appareil 310 touche accidentellement la substance ainsi recondensée est donc appréciablement limité.

En variante, la paroi périphérique 64 du récipient de stockage 60 peut n'être entourée que partiellement par la paroi de séparation interne 336 ; il est toutefois préférable dans ce cas que la surface de la paroi périphérique 64 non entourée par la paroi de séparation interne 336 soit limitée, afin de limiter la recondensation de la substance évaporée sur la paroi périphérique 64.

La paroi de séparation interne 336 est à demeure dans le boîtier 30. Dans l'exemple représenté, la paroi de séparation interne 336 reliée à la paroi interne 35 via une ou plusieurs nervures 339, dont l'une est visible sur la [Fig.8].

La paroi supérieure 32 et la paroi interne 35 sont formées dans une portion supérieure 30C du boîtier 30. Cette portion supérieure 30C est montée de façon coulissante de façon à pouvoir coulisser par rapport à la paroi latérale 39 suivant la direction descendante D. Plus précisément, la portion supérieure 30C est montée de façon à pouvoir coulisser entre :
- une position relevée dans laquelle la paroi supérieure 66 du récipient de stockage 60 est située au-delà de la paroi supérieure 32 suivant la direction descendante D lorsque le récipient de stockage 60 est reçu dans le réceptacle 31 ; et
- une position abaissée dans laquelle la paroi supérieure 66 est située au-delà de la paroi supérieure 32 suivant la direction montante U lorsque le récipient de stockage 60 est reçu dans le réceptacle 31.

Ainsi, lorsque la portion supérieure 30C est dans la position relevée (représentée sur la [Fig.8]), le récipient de stockage 60 peut être difficilement accessible voire inaccessible pour un utilisateur de l'appareil 310, tandis que le coulissement de la portion supérieure 30C vers la position abaissée rend le récipient de stockage 60 plus aisément accessible pour l'utilisateur. La portion supérieure 30C peut ainsi améliorer la sécurité d'utilisation de l'appareil 310.

Optionnellement, le boîtier 30 peut en outre comporter un dispositif de blocage mécanique, par exemple à cliquet, pour bloquer la portion supérieure 30C dans la position abaissée, et/ou un dispositif de rappel, par exemple à ressort, pour ramener la portion supérieure 30C dans la position relevée.

La ou les nervures 339 peuvent être solidaires de la paroi interne 35 de sorte que la paroi de séparation interne 336 est solidaire de la portion supérieure 30C et coulisse donc ensemble avec la portion supérieure 30C. En variante, la paroi de séparation interne 336 peut ne pas coulisser ensemble avec la portion supérieure 30C ; les nervures 339 peuvent alors être reçues dans des encoches (non représentées) que présente la paroi interne 35, ces encoches présentant un jeu correspondant à la distance de parcours de la portion supérieure 30C entre la position relevée et la position abaissée.

En variante, la portion supérieure 30C peut être non coulissante par rapport à la paroi latérale 39.

L'ensemble amovible 50 peut être fixé au boîtier 30 dans le réceptacle 31 via la paroi de séparation interne 336.

Dans l'exemple représenté, la paroi périphérique 64 du récipient de stockage 60 présente une saillie 68, et la saillie 68 est reçue dans une rainure en regard que présente la paroi de séparation interne 336. La saillie 68 et la rainure sont conformées de telle sorte que la coopération de la saillie 68 avec la rainure permet de fixer le récipient de stockage 60 par vissage ou par une fixation de type baïonnette, par exemple. Bien entendu, une telle fixation par vissage ou par fixation de type baïonnette peut être mise en œuvre de diverses manières, par exemple au moyen de plusieurs saillies et rainures correspondantes. Le récipient de stockage 60 peut aussi être fixé à la paroi de séparation interne 336 d'autres façons, par exemple par clipsage ou encliquetage.

En tout état de cause, le récipient de stockage 60 est ainsi fixé à la paroi de séparation interne 336 alors que la mèche 70 est déjà liée au récipient de stockage 60. En d'autres termes, l'ensemble amovible 50 est inséré d'un seul tenant dans le réceptacle 31, et le récipient de stockage 60 est fixé à la paroi de séparation interne 336 pendant l'insertion de l'ensemble amovible 50 dans le réceptacle 31. La paroi de séparation interne 336 définit à son extrémité inférieure une ouverture permettant le passage de la mèche 70 jusqu'à la position représentée sur la [Fig.8].

Dans l'exemple représenté, la mèche 70 présente une collerette 79 de diamètre supérieur à l'orifice de vidange 61, tandis que la portion principale 72 de la mèche 70 présente une section égale ou très légèrement inférieure à la section de l'orifice de vidange 61. La mèche 70 est insérée dans le récipient de stockage 60 depuis une extrémité supérieure ouverte de celui-ci, après quoi le récipient de stockage 60 est rempli de substance et fermé par un couvercle 66L, le couvercle 66L définissant la paroi supérieure 66 du récipient de stockage 60. En outre, un élément de maintien (non représenté) peut être prévu pour maintenir la mèche 70 en place dans le récipient de stockage 60 dans la position représentée dans la [Fig.8]. Dans un exemple, l'élément de maintien prend la forme d'une bague ou d'une pièce analogue qui est insérée en force dans le récipient de stockage 60 par son extrémité supérieure ouverte, jusqu'à ce que la bague appuie sur la mèche 70, avant de fermer le récipient de stockage 60 par le couvercle 66L. Dans un autre exemple, l'élément de maintien prend la forme d'une entretoise s'étendant depuis le couvercle 66L vers la mèche 70 et dont une extrémité appuie sur la mèche 70 lorsque le couvercle 66L ferme le récipient de stockage. Dans encore un autre exemple, l'élément de maintien prend la forme d'un godron, c'est-à-dire d'une saillie formée dans la paroi périphérique 64 du récipient de stockage 60 et faisant saillie dans le volume intérieur 69 du récipient de stockage 60. Avant de fermer le récipient de stockage 60 par le couvercle 66L, la mèche 70 est insérée en force de façon à franchir le godron. Une fois que la mèche 70 a franchi le godron, le godron s'oppose à un franchissement du godron par la mèche 70 en sens inverse et maintient ainsi la mèche 70 en place. Un ou plusieurs joints d'étanchéité (non représentés) peuvent être disposés de façon à garantir une liaison étanche entre la mèche 70 et l'orifice de vidange 61 et/ou une fermeture étanche du couvercle 66L.

En variante, il est aussi envisageable que les différentes liaisons de la mèche 70 au récipient de stockage 60 décrites ci-dessus soient mises en œuvre dans l'appareil 310.

Le fonctionnement de l'appareil 310 est identique à celui de l'appareil 10 et de l'appareil 210 et n'est donc pas décrit en détail à nouveau.

Il est envisageable que la paroi de séparation interne 336 soit mise en œuvre dans l'appareil 10 ou dans l'appareil 210. Il est aussi envisageable que la portion supérieure 30C, coulissante ou non, du boîtier 30, soit mise en œuvre dans l'appareil 10 ou dans l'appareil 210. Il est aussi envisageable que la liaison de la mèche 70 au récipient de stockage 60 au moyen de la collerette 79 soit mise en œuvre dans l'appareil 10 ou dans l'appareil 210.

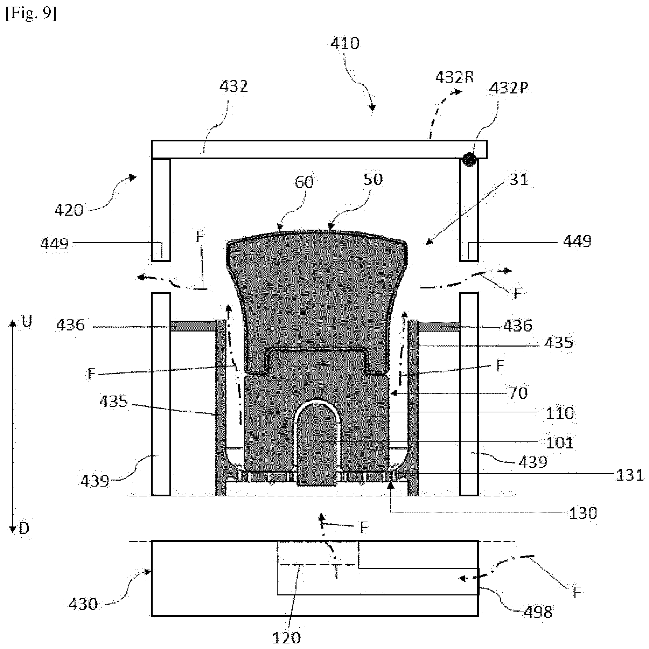

Une quatrième variante de réalisation d'un appareil 410 est représentée schématiquement en coupe sur la [Fig.9]. Sur cette figure, les éléments identiques à ceux décrits plus haut en rapport avec les figures 1 à 5C portent des références identiques et ne sont pas décrits en détail à nouveau.

La partie fixe 420 de l'appareil 410 comporte un bâti 430, qui est partiellement représenté sur la [Fig.9]. Le réceptacle 31 est aménagé dans une portion creuse du bâti 430, par exemple dans une portion creuse à l'extrémité supérieure du bâti 430.

De façon non représentée, le bâti 430 peut prendre un très grand nombre de formes, par exemple la forme d'une colonne reposant sur le sol via un pied, d'une colonne ancrée au sol, etc.

Dans l'exemple représenté, le réceptacle 31 est à l'extrémité supérieure du bâti 430, et le réceptacle 31 est délimité par une paroi périphérique 439 du bâti 430, une paroi supérieure 432 du bâti 430, et des parois 435, 436 internes au bâti 430. Les parois internes 435, 436 supportent ou sont formées d'un seul tenant avec la pièce de guidage d'air 130. De façon non représentée, la paroi périphérique 439 peut présenter une section horizontale circulaire, rectangulaire, ou autre.

La paroi supérieure 432 peut être montée à pivot par rapport à la paroi périphérique 439 au moyen d'un pivot 432P, de sorte que la paroi supérieure 432 peut être pivotée (cf. 432R sur la [Fig.9]) pour découvrir une ouverture supérieure délimitée par la paroi périphérique 439 et permettant l'insertion de l'ensemble amovible 50 dans le réceptacle 31.

Comme représenté schématiquement sur la [Fig.9], le bâti 430 présente une entrée d'air 498 en-dessous de la mèche 70. Par exemple l'entrée d'air 498 peut être à une extrémité inférieure du bâti 430. Le ventilateur 120 (représenté schématiquement en pointillés sur la [Fig.9]) est ici situé dans le bâti 430 entre l'entrée d'air 498 et la pièce de guidage d'air 130 suivant la direction descendante D. En variante, l'entrée d'air 498 peut être reliée à une canalisation d'air ou une autre arrivée d'air ; et le ventilateur 120 peut être déporté dans cette canalisation ou arrivée d'air.

Une ouverture 449 est pratiquée dans tout ou partie de la paroi périphérique 439 au-dessus de la mèche 70 suivant la direction montante U. Par exemple, comme représenté, l'ouverture 449 est en regard du récipient de stockage 60.

En fonctionnement, le ventilateur 120 amène un débit d'air depuis l'entrée d'air 498 vers les surfaces d'évaporation constituées par les parois extérieures de la mèche, puis vers l'ouverture 449. Les flèches F en trait mixte sur la [Fig.9] indiquent le sens et la direction de ce débit d'air. Ainsi, lorsque le ventilateur 120 et la résistance électrique 110 fonctionnent, l'appareil 410 génère un débit d'air chargé en substance évaporée, de débit d'air sortant par l'ouverture 449. En d'autres termes, l'ouverture 449 forme ici la sortie d'air unique du système d'aération de l'appareil 410.

Selon des variantes non représentées, l'ouverture 449 peut être subdivisée en plusieurs ouvertures formant chacune une sortie d'air, et/ou une ou plusieurs sorties d'air supplémentaires peuvent être formées dans la paroi supérieure 432.

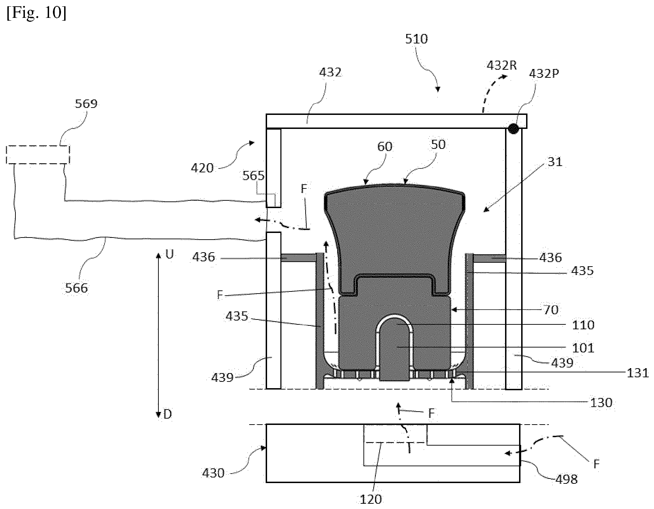

Une cinquième variante de réalisation d'un appareil 510 est représentée schématiquement en coupe sur la [Fig.10]. Sur cette figure, les éléments identiques à ceux décrits plus haut portent des références identiques et ne sont pas décrits en détail à nouveau.

L'appareil 510 diffère de l'appareil 410 en cela que la sortie d'air du système d'aération est déportée par rapport au bâti 430. Plus concrètement, une ouverture de communication 565 est pratiquée dans la paroi périphérique 439 et met en communication le réceptacle 31 avec une canalisation de sortie 566. La canalisation de sortie 566 met en communication fluide le réceptacle 31, via l'ouverture de communication 565, avec une sortie d'air 569.

En fonctionnement, le ventilateur 120 amène un débit d'air depuis l'entrée d'air 498 vers les surfaces d'évaporation constituées par les parois extérieures de la mèche, puis vers l'ouverture de communication 565, puis vers la sortie d'air 569 via la canalisation de sortie 566. Les flèches F en trait mixte sur la [Fig.10] indiquent le sens et la direction de ce débit d'air. Ainsi, lorsque le ventilateur 120 et la résistance électrique 110 fonctionnent, l'appareil 510 génère un débit d'air chargé en substance évaporée, de débit d'air sortant par la sortie d'air 569. En d'autres termes, la sortie d'air 569 forme ici la sortie d'air unique du système d'aération de l'appareil 510, cette sortie d'air pouvant être à une certaine distance de l'ensemble amovible 50. Ainsi, l'appareil 510 peut permettre de diffuser un débit d'air chargé en substance évaporée à une certaine distance de l'ensemble amovible 50.

La sortie d'air 569 peut prendre un très grand nombre de formes, par exemple la forme d'un embout, d'un orifice grillagé, etc. La sortie d'air 569 peut avoir une orientation quelconque et/ou être protégée par un élément de protection non représenté, tant que celui-ci permet au débit d'air de sortir par la canalisation de sortie 566 tel que décrit.

Selon des variantes non représentées, plusieurs canalisations de sortie 566 peuvent être prévues, chacune de ces canalisations de sortie 566 étant associée à une ouverture de communication 565 à une sortie d'air 569, et/ou une ou plusieurs sorties d'air supplémentaires peuvent être formées dans la paroi supérieure 432.

Il est envisageable que la paroi de séparation interne 336 et/ou les différentes liaisons de la mèche 70 au récipient de stockage 60 décrites ci-dessus soient mises en œuvre dans l'appareil 410 ou dans l'appareil 510.

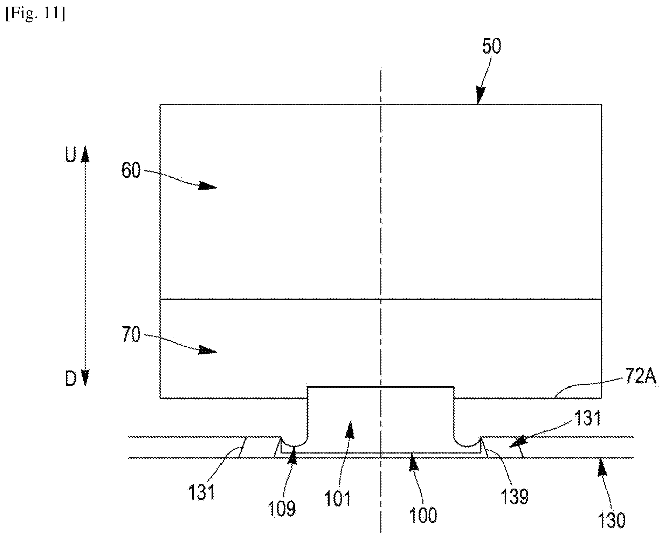

La [Fig.11] est une vue schématique en coupe plane d'une variante d'exécution de la pièce de guidage d'air 130, cette variante d'exécution pouvant être mise en œuvre dans toutes les variantes de réalisation de l'appareil décrites jusqu'ici. Les éléments identiques à ceux décrits plus haut portent des références identiques et ne sont pas décrits en détail à nouveau. Les éléments représentés sur la [Fig.11] ne sont représentés que schématiquement à des fins d'explication.

Dans cette variante d'exécution, l'organe de chauffage 100 présente, par exemple au niveau de sa tige 101, une dépression 109 sous la surface inférieure 72A de la mèche 70 suivant la direction descendante D. La dépression 109 présente une concavité tournée vers la surface inférieure 72A de la mèche 70.

Par conséquent, s'il se produit un gouttage de la substance à l'état liquide au niveau de la mèche 70 du fait du chauffage de la mèche 70 par l'organe de chauffage 100, la substance ayant ainsi goutté s'accumule dans la dépression 109. La substance est ainsi empêchée de goutter vers d'autres éléments de l'appareil, comme le ventilateur 120 par exemple. D'autre part, la substance accumulée dans la dépression 109 peut s'évaporer du fait de sa température relativement élevée et/ou par l'action du débit d'air généré par le ventilateur 120. La substance ayant goutté peut donc être évaporée puis évacuée.

La tige 101 et/ou l'organe de guidage d'air 130 peuvent être conformés de diverses façons de façon à favoriser l'écoulement de la substance ayant goutté vers la dépression 109, par exemple avec une légère inclinaison descendante en direction de la dépression 109. D'autre part, les trous traversants 131 peuvent être disposés à une distance minimale de la dépression 109, par exemple 3 mm ou plus, de façon à éviter que la substance ayant goutté ne passe par les trous traversants.

Selon des variantes non représentées, la dépression 109 est formée dans l'organe de guidage d'air 130 et non dans l'organe de chauffage 100, ou encore l'organe de guidage d'air 130 et l'organe de chauffage 100 présentent chacun une dépression 109.

On a décrit jusqu'ici des modes de réalisation où la substance est à l'état liquide à température ambiante. Toutefois, en variante, la substance est à l'état solide à température ambiante. Par exemple la substance peut présenter à la pression atmosphérique une température de fusion supérieure à 30°C, par exemple comprise entre 30°C et 40°C. Dans ce cas, le récipient de stockage 60 contient la substance à l'état solide à température ambiante. Le chauffage de la mèche 70 par l'organe de chauffage 100 provoque une fusion locale de la substance au voisinage de l'orifice de vidange 61. La substance ainsi devenue liquide peut alors s'écouler à travers la mèche 70 ou comme on l'a décrit plus haut.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

## Revendications

1. Appareil diffuseur (10 ; 210 ; 310 ; 410, 510) pour disperser dans l'air, à l'état de vapeur, une substance à l'état liquide ou solide à température ambiante, l'appareil diffuseur comportant :
- un réceptacle (31) ;
- un récipient de stockage (60) contenant la substance et présentant un orifice de vidange (61), le récipient de stockage (60) étant reçu dans le réceptacle (31) ;
- un organe distributeur positionné en sortie de l'orifice de vidange (61) et relié à l'orifice de vidange (61), l'organe distributeur comprenant un corps poreux (70) présentant une surface d'évaporation située dans le réceptacle (31), le récipient de stockage (60) étant reçu dans le réceptacle (31) de sorte que l'orifice de vidange (61) est orienté vers le corps poreux (70) suivant une direction descendante (D) de l'appareil diffuseur, et le corps poreux (70) étant situé au-delà de l'orifice de vidange (61) suivant la direction descendante (D) de l'appareil diffuseur ;
- un organe de chauffage (100) agencé sur ou dans le corps poreux (70) de manière à contrôler un écoulement de la substance à travers le corps poreux (70) ; et
- un système d'aération comprenant une entrée d'air (98), au moins un ventilateur (120) et au moins une sortie d'air (33 ; 449 ; 569), le système d'aération étant configuré pour créer un débit d'air allant depuis l'entrée d'air (98) vers ladite au moins une sortie d'air en balayant la surface d'évaporation dans une direction montante (U) de l'appareil diffuseur (10 ; 210 ; 310 ; 410, 510) opposée à la direction descendante (D),
l'appareil diffuseur étant destiné à être utilisé dans une position d'utilisation dans laquelle la direction descendante (D) est vers le bas et la direction montante (U) est vers le haut par rapport à l'accélération de la pesanteur,
dans lequel la substance est une substance liquide qui présente une viscosité variable en fonction de la température, ladite viscosité étant telle que, dans la position d'utilisation, la substance ne peut pas s'écouler à travers le corps poreux (70) à toute température ambiante inférieure à une première température, la première température étant supérieure à 0°C, et que la substance s'écoule à travers le corps poreux (70) à une deuxième température supérieure à la première température.

2. Appareil diffuseur (10 ; 210 ; 310) selon la revendication 1, dans lequel le réceptacle (31) débouche à l'air libre par une ouverture (33) orientée dans la direction montante (U) de l'appareil diffuseur, l'ouverture (33) formant la ou une dite sortie d'air du système d'aération.

3. Appareil diffuseur (10 ; 210 ; 310) selon la revendication 2, dans lequel l'orifice de vidange (61) est disposé à une première extrémité du récipient de stockage (60), et dans lequel le récipient de stockage (60) présente, à une deuxième extrémité opposée à la première extrémité, une portion divergente (62, 63) facilitant la préhension du récipient de stockage et disposée au-delà de l'ouverture (33) suivant la direction montante (U) de l'appareil diffuseur de façon à dévier le flux d'air sortant de l'ouverture (33) vers une périphérie de l'appareil diffuseur.

4. Appareil diffuseur (10 ; 210 ; 310 ; 410 ; 510) selon l'une quelconque des revendications 1 à 3, dans lequel le ventilateur (120) est disposé au-delà du corps poreux (70) suivant la direction descendante (D) de l'appareil diffuseur.

5. Appareil diffuseur (10 ; 210 ; 310 ; 410 ; 510) selon l'une quelconque des revendications 1 à 4, dans lequel l'entrée d'air (98) est au-delà du corps poreux (70) suivant la direction descendante (D) de l'appareil diffuseur.

6. Appareil diffuseur (10 ; 210 ; 310 ; 410 ; 510) selon l'une quelconque des revendications 1 à 5, dans lequel le système d'aération comprend en outre un organe de guidage d'air (130), l'organe de guidage d'air (130) comportant au moins un trou traversant (131) en regard de la surface d'évaporation.

7. Appareil diffuseur (10 ; 210 ; 310 ; 410 ; 510) selon l'une quelconque des revendications 1 à 6, dans lequel l'organe de chauffage (100) comprend une résistance électrique (110) placée directement sur une surface extérieure du corps poreux (70) ou reçue en partie dans un évidement (75), de préférence aveugle, que présente le corps poreux (70).

8. Appareil diffuseur (10 ; 210 ; 310 ; 410 ; 510) selon les revendications 6 et 7 prises en combinaison, dans lequel l'organe de chauffage (100) s'étend à travers l'organe de guidage d'air (130).

9. Appareil diffuseur (10 ; 210 ; 310 ; 410 ; 510) selon la revendication 7 ou la revendication 8, comprenant en outre une carte électronique (150), la résistance électrique (110) étant alimentée électriquement par la carte électronique (150).

10. Appareil diffuseur (10 ; 210 ; 310 ; 410 ; 510) selon l'une quelconque des revendications 1 à 9, comprenant en outre un dispositif de commande configuré pour commander l'organe de chauffage en fonction d'une température de consigne dans le corps poreux (70).

11. Appareil diffuseur (10 ; 210 ; 310 ; 410 ; 510) selon la revendication 9 et la revendication 10 prises en combinaison, dans lequel le dispositif de commande est agencé sur la carte électronique (150).

12. Appareil diffuseur (10 ; 210 ; 310 ; 410 ; 510) selon la revendication 10 ou selon la revendication 11, dans lequel le dispositif de commande est en outre configuré pour commander une vitesse de fonctionnement du ventilateur (120).

13. Appareil diffuseur (10 ; 210 ; 310 ; 410 ; 510) selon l'une quelconque des revendications 1 à 12, dans lequel la substance comporte au moins un composé pris dans le groupe formé par les agents odorifères utilisables pour l'homme ou l'animal, les substances sémiochimiques, les agents cosmétiques, les huiles essentielles, les parfums, les agents désinfectants, les agents neutralisants d'odeur et les agents phytosanitaires et agricoles.

14. Appareil diffuseur (10 ; 210 ; 310 ; 410 ; 510) selon la revendication 13, dans lequel la substance comporte au moins un composé pris dans le groupe formé par les agents odorifères utilisables pour l'homme, les agents cosmétiques, les huiles essentielles, les parfums, les agents désinfectants, les agents neutralisants d'odeur.

15. Utilisation d'un appareil diffuseur (10 ; 210 ; 310 ; 410 ; 510) selon l'une quelconque des revendications 1 à 14 pour disperser dans l'air, à l'état de vapeur, une substance à l'état liquide ou solide à température ambiante, dans laquelle l'appareil diffuseur est dans la position d'utilisation, la direction descendante (D) de l'appareil diffuseur étant vers le bas par rapport à l'accélération de pesanteur.

16. Utilisation selon la revendication 15, dans laquelle l'appareil diffuseur (10 ; 210 ; 310 ; 410 ; 510) est situé dans un lieu clos ou dans un lieu à l'abri des précipitations.

## Patentansprüche

1. Diffusionsvorrichtung (10; 210; 310; 410, 510) zum Verteilen einer bei Raumtemperatur flüssigen oder festen Substanz in Form von Dampf in der Luft, wobei die Diffusionsvorrichtung umfasst:
- einen Behälter (31);
- einen die Substanz enthaltenden Vorratsbehälter (60) umfassend eine Entleerungsöffnung (61), wobei der Vorratsbehälter (60) in dem Behälter (31) aufgenommen ist;
- ein am Ausgang der Entleerungsöffnung (61) angeordnetes und mit dieser verbundenes Abgabeteil, das einen porösen Körper (70) mit einer im Behälter (31) vorhandenen Verdunstungsfläche umfasst, wobei der Vorratsbehälter (60) in dem Behälter (31) so aufgenommen ist, dass die Entleerungsöffnung (61) in Abwärtsrichtung (D) der Diffusionsvorrichtung zum porösen Körper (70) hin ausgerichtet ist, und der poröse Körper (70) in Abwärtsrichtung (D) der Diffusionsvorrichtung über der Entleerungsöffnung (61) angeordnet ist;
- eine Heizvorrichtung (100), die an oder in dem porösen Körper (70) angeordnet ist, um einen Durchfluss der Substanz durch den porösen Körper (70) zu steuern; und
- ein Belüftungssystem umfassend einen Lufteinlass (98), mindestens einen Ventilator (120) und mindestens einen Luftauslass (33; 449; 569), wobei das Belüftungssystem so konfiguriert ist, dass es einen Luftstrom von dem Lufteinlass (98) zu dem mindestens einen Luftauslass erzeugt, indem es die Verdunstungsfläche in einer Aufwärtsrichtung (U) der Diffusionsvorrichtung (10; 210; 310; 410, 510) zur Abwärtsrichtung (D) entgegensetzt,
wobei die Diffusionsvorrichtung dazu bestimmt ist, in einer Gebrauchsstellung verwendet zu werden, in der die Abwärtsrichtung (D) nach unten und die Aufwärtsrichtung (U) nach oben in Bezug auf die Erdbeschleunigung ist,
wobei die Substanz eine flüssige Substanz ist, die eine temperaturabhängige Viskosität aufweist, wobei die Viskosität derart ist, dass in der Gebrauchsstellung die Substanz bei jeder Umgebungstemperatur unterhalb einer ersten Temperatur nicht durch den porösen Körper (70) fließen kann, wobei die erste Temperatur über 0°C liegt, und die Substanz bei einer zweiten Temperatur über der ersten Temperatur durch den porösen Körper (70) fließt.

2. Diffusionsvorrichtung (10; 210; 310) gemäß Anspruch 1, wobei der Behälter (31) durch eine in Aufwärtsrichtung (U) der Diffusionsvorrichtung ausgerichtete Öffnung (33) ins Freie mündet, wobei die Öffnung (33) den oder einen Luftausgang des Belüftungssystems bildet.

3. Diffusionsvorrichtung (10; 210; 310) gemäß Anspruch 2, wobei die Entleerungsöffnung (61) an einem ersten Ende des Vorratsbehälters (60) angeordnet ist und wobei der Vorratsbehälter (60) an einem zweiten Ende, das dem ersten Ende gegenüberliegt, einen divergierenden Abschnitt (62, 63) aufweist, der das Greifen des Vorratsbehälters erleichtert und über die Öffnung (33) in Aufwärtsrichtung (U) der Diffusionsvorrichtung hinaus angeordnet ist, um den aus der Öffnung (33) austretenden Luftstrom zu einem Umfang der Diffusionsvorrichtung umzuleiten.

4. Diffusionsvorrichtung (10; 210; 310; 410; 510) gemäß einem der Ansprüche 1 bis 3, wobei der Ventilator (120) in Abwärtsrichtung (D) der Diffusionsvorrichtung hinter dem porösen Körper (70) angeordnet ist.

5. Diffusionsvorrichtung (10; 210; 310; 410; 510) gemäß einem der Ansprüche 1 bis 4, wobei der Lufteinlass (98) in Abwärtsrichtung (D) der Diffusionsvorrichtung unter dem porösen Körper (70) liegt.

6. Diffusionsvorrichtung (10; 210; 310; 410; 510) gemäß einem der Ansprüche 1 bis 5, wobei das Belüftungssystem außerdem ein Luftleitelement (130) umfasst, wobei das Luftleitelement (130) mindestens ein Durchgangsloch (131) gegenüber der Verdunstungsfläche umfasst.

7. Diffusionsvorrichtung (10; 210; 310; 410; 510) gemäß einem der Ansprüche 1 bis 6, wobei das Heizelement (100) einen elektrischen Widerstand (110) umfasst, der direkt auf einer Außenfläche des porösen Körpers (70) angeordnet oder teilweise in einer Aussparung (75) aufgenommen ist, vorzugsweise blind, die der poröse Körper (70) aufweist.

8. Diffusionsvorrichtung (10; 210; 310; 410; 510) gemäß den Ansprüchen 6 und 7 in Kombination, wobei sich das Heizelement (100) durch das Luftleitelement (130) erstreckt.

9. Diffusionsvorrichtung (10; 210; 310; 410; 510) gemäß Anspruch 7 oder Anspruch 8, umfassend zusätzlich eine elektronische Karte (150), wobei der elektrische Widerstand (110) von der elektronischen Karte (150) mit Strom versorgt wird.

10. Diffusionsvorrichtung (10; 210; 310; 410; 510) gemäß einem der Ansprüche 1 bis 9, umfassend außerdem eine Steuervorrichtung, die so konfiguriert ist, dass sie das Heizelement entsprechend einer Solltemperatur im porösen Körper (70) steuert.

11. Diffusionsvorrichtung (10; 210; 310; 410; 510) gemäß Anspruch 9 und Anspruch 10 in Kombination, wobei die Steuervorrichtung auf der elektronischen Karte (150) angeordnet ist.

12. Diffusionsvorrichtung (10; 210; 310; 410; 510) gemäß Anspruch 10 oder gemäß Anspruch 11, wobei die Steuervorrichtung ferner dazu konfiguriert ist, eine Betriebsgeschwindigkeit des Ventilators (120) zu steuern.

13. Diffusionsvorrichtung (10; 210; 310; 410; 510) gemäß einem der Ansprüche 1 bis 12, wobei die Substanz mindestens eine Verbindung aus der Gruppe bestehend aus für Menschen oder Tiere verwendbaren Duftstoffen, Semio-chemischen Substanzen, kosmetischen Wirkstoffen, ätherischen Ölen, Duftstoffen, Desinfektionsmitteln, Geruchsneutralisatoren und Pflanzenschutz- und Agrarchemikalien umfasst.

14. Diffusionsvorrichtung (10; 210; 310; 410; 510) gemäß Anspruch 13, wobei die Substanz mindestens eine Verbindung aus der Gruppe bestehend aus für den Menschen verwendbaren Duftstoffen, kosmetischen Wirkstoffen, ätherischen Ölen, Parfüms, Desinfektionsmitteln und Geruchsneutralisatoren umfasst.

15. Verwendung einer Diffusionsvorrichtung (10; 210; 310; 410; 510) gemäß einem der Ansprüche 1 bis 14 zum Verteilen einer bei Raumtemperatur flüssigen oder festen Substanz in Form von Dampf in der Luft, wobei sich die Diffusionsvorrichtung in der Gebrauchsstellung ist und die Abwärtsrichtung (D) der Diffusionsvorrichtung in Bezug auf die Erdbeschleunigung nach unten verläuft.

16. Verwendung gemäß Anspruch 15, wobei die Diffusionsvorrichtung (10; 210; 310; 410; 510) an einem geschlossenen Ort oder an einem Ort, der vor Niederschlägen geschützt ist, angeordnet ist.

## Claims

1. A diffusing apparatus (10; 210; 310; 410, 510) for dispersing into the air in the vapor state a substance in the liquid or solid state at ambient temperature, the diffusing apparatus including:
- a receptacle (31);
- a storage container (60) for containing the substance and including a drain orifice (61), the storage container (60) being received in the receptacle (31) so that the drain orifice (61) is oriented in a downward direction (D) of the diffusing apparatus;
- a dispensing member positioned at the outlet of the drain orifice (61) and connected to the drain orifice (61), the dispensing member comprising a porous body (70) having an evaporation surface situated in the receptacle (31) and being situated beyond the drain orifice (61) in the downward direction (D) of the diffusing apparatus;
- a heating member (100) arranged on or in the porous body (70) so as to control a flow of the substance through the porous body (70); and
- an aeration system comprising an air intake (98), at least one fan (120) and at least one air outlet (33; 449; 569), the aeration system being configured to create a flow of air from the air intake (98) to said at least one air outlet and sweeping the evaporation surface in an upward direction (U) of the diffusing apparatus (10; 210; 310; 410, 510) opposite the downward direction (D),
the diffusing apparatus being intended to be used in a position of use in which the downward direction (D) is toward the bottom and the upward direction (U) is toward the top relative to the acceleration due to gravity,
wherein the substance is a liquid substance that has a viscosity variable as a function of temperature, said viscosity being such that in the position of use the substance cannot flow through the porous body (70) at any ambient temperature below a first temperature, the first temperature being above 0°C, and the substance flows through the porous body (70) at a second temperature above the first temperature..

2. The diffusing apparatus (10; 210; 310) as claimed in claim 1 in which the receptacle (31) discharges into the open air via an opening (33) oriented in the upward direction (U) of the diffusing apparatus, the opening (33) forming the air outlet or one of said air outlets of the aeration system.

3. The diffusing apparatus (10; 210; 310) as claimed in claim 2 in which the drain orifice (61) is at a first end of the storage container (60) and the storage container (60) has at a second end opposite the first end a divergent portion (62, 63) facilitating holding the storage container and beyond the opening (33) in the upward direction (U) of the diffusing apparatus so as to divert the flow of air exiting the opening (33) to a periphery of the diffusing apparatus.

4. The diffusing apparatus (10; 210; 310; 410; 510) as claimed in any one of claims 1 to 3 in which the fan (120) is beyond the porous body (70) in the downward direction (D) of the diffusing apparatus.

5. The diffusing apparatus (10; 210; 310; 410; 510) as claimed in any one of claims 1 to 4 in which the air intake (98) is beyond the porous body (70) in the downward direction (D) of the diffusing apparatus.

6. The diffusing apparatus (10; 210; 310; 410; 510) as claimed in any one of claims 1 to 5 in which the aeration system further includes an air guide member (130), the air guide member (130) including at least one through-hole (131) facing the evaporation surface.

7. The diffusing apparatus (10; 210; 310; 410; 510) as claimed in any one of claims 1 to 6 in which the heating member (100) comprises an electrical resistance (110) placed directly on an exterior surface of the porous body (70) or received in part in an opening (75), preferably a blind opening, in the porous body (70).

8. The diffusing apparatus (10; 210; 310; 410; 510) as claimed in claims 6 and 7 in conjunction in which the heating member (100) extends through the air guide member (130).

9. The diffusing apparatus (10; 210; 310; 410; 510) as claimed in claim 7 or claim 8 further comprising an electronic circuit card (150), the electrical resistance (110) being supplied with electricity by the electronic circuit card (150).

10. The diffusing apparatus (10; 210; 310; 410; 510) as claimed in any one of claims 1 to 9 further comprising a control device configured to control the heating member as a function of a setpoint temperature in the porous body (70).

11. The diffusing apparatus (10; 210; 310; 410; 510) as claimed in claim 9 and claim 10 in conjunction in which the control device is on the electronic circuit card (150).

12. The diffusing apparatus (10; 210; 310; 410; 510) as claimed in claim 10 or claim 11 in which the control device is further configured to control an operating speed of the fan (120).

13. The diffusing apparatus (10; 210; 310; 410; 510) as claimed in any one of claims 1 to 12 in which the substance includes at least one compound selected from the group formed by odoriferous agents usable on man or on animals, semiochemical substances, cosmetic agents, essential oils, perfumes, disinfectants, odor neutralizing agents and phytosanitary and agricultural agents.

14. The diffusing apparatus (10; 210; 310; 410; 510) as claimed in claim 13 in which the substance includes at least one compound selected from the group formed by odoriferous agents usable on man, cosmetic agents, essential oils, perfumes, disinfectants, odor neutralizing agents.

15. Use of a diffusing apparatus (10; 210; 310; 410; 510) as claimed in any one of claims 1 to 14 for dispersing into the air in the vapor state a substance in the liquid or solid state at ambient temperature in which the diffusing apparatus is in the position of use, the downward direction (D) of the diffusing apparatus being toward the bottom relative to the acceleration due to gravity.

16. Use as claimed in claim 15 in which the diffusing apparatus (10; 210; 310; 410; 510) is in a closed space or in a place sheltered from precipitation.
